# EUROPEAN PATENT SPECIFICATION

(11) **EP 2 250 497 B1**
(45) Date of publication and mention of the grant of the patent: **10.09.2014**
(21) Application number: 09712569.4
(22) Date of filing: 18.02.2009
(51) Int. Cl.: C12N 5/071

(54) **CELL PROCESSING AND/OR ENRICHMENT METHODS**
ZELLPROZESSIERUNGS- UND/ODER -ANREICHERUNGSVERFAHREN
PROCEDES DE TRAITEMENT ET/OU D'ENRICHISSEMENT DE CELLULES

(30) Priority: 18.02.2008 US 29496 P; 03.07.2008 US 78230 P
(43) Date of publication of application: 17.11.2010
(73) Proprietor: Genetic Technologies Limited, Fitzroy, VIC 3065 (AU)
(72) Inventor: MANTZARIS, Debbie, Avondale Heights Victoria 3034 (AU); ALLMAN, Richard, Wyndham Vale Victoria 3124 (AU)
(74) Representative: Harding, Charles Thomas
(86) International application number: PCT/AU2009/000180
(87) International publication number: WO 2009/103110

(56) References cited:
- WO-A1-2005/047532
- WO-A1-2005/047532
- WO-A1-2006/119569
- US-A- 4 862 899
- ADINOLFI MATTEO ET AL: "Fetal cells in transcervical samples at an early stage of gestation", JOURNAL OF HUMAN GENETICS, vol. 46, no. 3, 2001, pages 99-104, XP002641944, ISSN: 1434-5161

## Description

### FIELD OF THE INVENTION

The present invention relates to methods of processing and/or enriching cells from a pregnant female. More particularly, the invention provides methods for processing and/or enriching fetal cells from a pregnant female. The fetal cells can be used in a variety of procedures including, detection of a trait of interest such as a disease trait, or a genetic predisposition thereto, gender typing and parentage testing.

### BACKGROUND OF THE INVENTION

Early prenatal diagnosis to detect fetal genetic disorders is desirable for both expectant mothers and physicians to make informed decisions. Definitive methods of invasive prenatal testing (amniocentesis and chorionic villous sampling) carry a small, but significant risk of miscarriage, and the results are rarely available before 13 weeks of pregnancy because of the time required for cell culture and analysis.

"Non-invasive" screening with maternal serum analyte screening and ultrasound can identify individuals at risk for fetal aneuploidy (predominantly trisomy 21), but a positive screening result still requires a subsequent invasive procedure for a definitive diagnosis. Of some 25-30 such procedures, only one will actually show a fetal aneuploidy.

Many laboratories around the world have been attempting for over a decade to develop non-invasive (i.e. venupuncture only) methods to isolate and analyse fetal cells. An obvious advantage is that definitive results can be obtained using molecular techniques such as fluorescence in-situ hybridization (FISH) and quantitative fluorescent polymerase chain reaction (QF-PCR) on recovered fetal cells.

The presence of fetal cells in maternal blood provides potentially the best possible source of cells for non-invasive prenatal diagnosis. However fetal cells are present at very low numbers, and their isolation is not a trivial task, with only 1 or 2 fetal cells being present per 10 ml maternal blood. Our evidence also indicates that the presence of intact fetal cells in the maternal circulation is not a universal event.

An attractive alternative to peripheral blood sampling is the isolation and analysis of trophoblasts from transcervical samples. Unlike maternal blood in which multiple circulating fetal cell types exist, fetal cells in the transcervical samples are all of placental origin and are overwhelmingly trophoblasts (Bischoff and Simpson, 2006).

It was long assumed that the cervical canal contained trophoblasts of fetal origin. The early embryo is covered with chorion levae, but later in the gestation the chorionic surface is smooth. However, it was not until 1971 that the presence of fetal cells in the endocervix was confirmed by identification of Y-chromosome bearing cells in midcervical mucous samples collected with a cotton swab (Shettles *et al.,* 1971). Subsequent reports assumed that these fetal cells were shed from the regressing chorionic villous into the lower uterine pole (Warren *et al.,* 1972, Adinolphi *et al.,* 1995a, Rhine *et al.,* 1975). In this scenario, shedding is most likely to occur between 7 and 13 weeks gestation, before fusion of the deciduas basalis and parietalis. Desquamated trophoblasts are believed first to accumulate behind the cervical mucous at the level of the internal opening section (Bulmer *et al.,* 1995, Adinolphi and Sherlock, 1997) and then become ensconced in the cervical mucous.

These biologic events thus define the window of opportunity for endocervical sampling to be of use for prenatal diagnoses, although several studies have demonstrated trophoblast recovery as early as 5 weeks gestation (Katz-Jaffe *et al.,* 2005, Mantzaris *et al.,* 2005).

Efforts to extract trophoblasts were first made in the 1970's. Rhine *et al.* (1975 and 1977) described "antenatal cell extractors" that flush the endocervical canal with sterile saline to recover fetal cells. After culture, fetal metaphases from recovered cells were detected in approximately 50% of cases. However, other investigators reported negative results (Goldberg *et al.,* 1980), leading to overall skepticism concerning clinical application. In hindsight, inability to detect fetal cells probably also reflected deficiencies in the clinicians' techniques in obtaining the endocervical specimen, as well as poor sensitivity of methods used to confirm the presence of fetal cells.

Interest was rekindled in the 1990's following the introduction of chorionic villus sampling. Transcervical specimens were collected by cotton swabs, cytobrush, aspiration of cervical mucus with a catheter, lavage of the endocervical canal or uterine. A variety of techniques resulted in detection of fetal cells in 40-90% of specimens examined (Adinolfi *et al.,* 1995a, Bussani *et al.,* 2002, Cioni *et al.,* 2003, Fejgin *et al.,* 2001, Massari *et al.,* 1996; Miller *et al.,* 1999; Rodeck *et al.,* 1995; Tuttschek *et al.,* 1995). Again, however, interest waned in most centres because analysis was difficult. The presumptive fetal cells embedded in mucous were not readily amenable to FISH. More recently, molecular PCR techniques for micromanipulated cell clumps of trophoblastic origin were demonstrated to have utility for transcervical samples (Bussani *et al.,* 2004; Bussani *et al.,* 2007; Katz-Jaffe *et al.,* 2005).

Most transcervical specimens contain a variety of maternally derived cells (leukocytes, macrophages, squamous epithelia, columnar epithelia, and endocervical cells) as well as different fetal-derived cells (cytotrophoblasts and syncytiotrophoblasts) (Bulmer *et al.,* 1995, Miller *et al.,* 1999). The frequency of each fetal cell type is variable and seemingly dependent on the collection method and gestational age.

The literature is inconsistent with regard to the number and relative proportion of fetal cells which can be recovered in transcervical specimens. Kingdom *et al.* (1995) reported the frequency of fetal XY cells recovered by endocervical lavage to range from 2 to 8%. In the same study, FISH results using a cytological brush ranged from 1 to 5% of total cells. Daryani *et al.* (1997) reported fetal cells to be 3.6 to 47.8% of total cells, based on 3-31 fetal cells obtained by aspiration. Katz-Jaffe *et al.* (2005) claimed a higher absolute number of fetal cells, up to 250 cells/ml. of dissociated mucous, based on immunohistochemistry staining with trophoblast specific monoclonal antibodies (NDOG1 and FT141.1).

WO 2005/047532 discloses a method of analysing the genetic characteristics of a reproductive cell sample taken from a subject. WO 2006/119569 discloses methods for selecting fetal cells based on MHC expression, telomerase expression and/or telomere length.

There is a need for alternate methods for processing and/or enriching fetal cells from a transcervical sample.

### SUMMARY OF THE INVENTION

Although it has been suggested in the art that transcervical samples are a poor source of fetal cells for genetic analysis, the present inventors have surprisingly developed a reliable and efficient method for processing transcervical samples. The cells obtained using this procedure can be used for genetic analysis, or subjected to selection procedures for enriching the fetal cells.

Accordingly, the present invention provides a method as defined in the appended claims.

Disclosed herein is a method for processing a transcervical sample from a pregnant female, the method comprising
i) treating the sample to produce at least a partial single cell suspension,
ii) selecting cells based on cell size,
wherein at least some of the cells obtained from step ii) are fetal cells which are suitable for genetic analysis and/or enrichment

The present inventors have surprisingly found that mechanically disaggregating the sample allows suitable numbers of fetal cells to beobtained. Thus, in a preferred embodiment step i) comprises treating the sample by at least partially mechanically disaggregating the sample. This means that enzymatic steps may not be required, significantly reducing costs. However, in an alternate embodiment step i) comprises treating the sample by at least partially enzymatically disaggregating the sample. In a further embodiment, the method comprises both mechanically and enzymatically disaggregating the sample.

In a preferred embodiment, step ii) comprises filtering the product of step i) through a cell strainer and collecting the cells that pass through the cell strainer. Preferably, the cell strainer has a mesh size of about 100µM.

In another embodiment, the method further comprises
iii) treating material which did not pass through the cell strainer with at least one enzyme capable of disassociating aggregated cells.

In addition, the method further comprises
iv) selecting cells obtained from step iii) based on cell size.

In an embodiment, the method further comprises pooling the cells obtained from step ii) with the cells obtained from step iv).

In an alternate embodiment, step ii) comprises flow cytometry cell sorting of unlabelled cells.

In another aspect, the present invention provides a method of enriching fetal cells from a transcervical sample from a pregnant female, the method comprising
i) processing a transcervical sample according to the method of any one of claims 1 to 11, and
ii) positively and/or negatively selecting fetal cells.

In a preferred embodiment, negatively selecting fetal cells comprises removing cells that express at least one MHC molecule on their surface.

In a preferred embodiment, positively selecting fetal cells comprises using an agent which binds syncytiotrophoblasts and/or cytotrophoblasts.

In another embodiment, positively selecting fetal cells comprises selecting cells that express telomerase and/or selecting cells based on telomere length. Preferably, selecting cells that express telomerase and/or selecting cells based on telomere length comprises detecting a protein component of telomerase, detecting an RNA component of telomerase and/or detecting an mRNA encoding a protein component of telomerase.

Whilst micromanipulation and/or laser microdissection can be used to isolate labelled cells these labour and time consuming procedures are not necessary. Thus, in a further preferred embodiment the method does not comprise micromanipulation or laser microdissection.

The present inventors have surprisingly found that enriching multinucleated fetal cells from a transcervical sample based on cell size can provide a sufficient purity and yield of fetal cells to allow for various prenatal diagnostics to be performed.

Thus, the present invention provides a method of enriching multinucleated fetal cells from a transcervical sample from a pregnant female, the method comprising selecting cells which are between about 40µm and 150µm in size.

In an embodiment of the above aspect, the cells are selected using at least two cell strainers with different mesh sizes, flow cytometry on unlabelled cells, microfluidics, or a combination thereof.

In another embodiment of the above aspect, the method comprises treating the sample to produce at least a partial single cell suspension before the cells are selected.

In a preferred embodiment of the above aspect, the method comprises
i) at least partially mechanically disaggregating the sample to produce at least a partial single cell suspension,
ii) filtering the at least partial single cell suspension through a first cell strainer which has a mesh size of about 100µM, and collecting the cells that pass through the first cell strainer, and
iii) filtering the cells collected in step ii) through a second cell strainer which has a mesh size of about 40µM, and collecting the cells that did not pass through the second cell strainer.

Cells that pass through the second strainer comprise non-multinucleated fetal cells which can be positively and/or negatively selected using a method described herein, and if desired, pooled with the multinucleated fetal cells.

In an alternate embodiment of the above aspect, the method comprises
i) at least partially enzymatically disaggregating the sample to produce at least a partial single cell suspension, and
ii) filtering the at least partial single cell suspension through a cell strainer which has a mesh size of about 40µM, and collecting the cells that did not pass through the cell strainer.

In a further alternate embodiment of the above aspect, the method comprises
i) at least partially mechanically disaggregating the sample to produce at least a partial single cell suspension,
ii) filtering the at least partial single cell suspension through a cell strainer which has a mesh size of about 100µM, and collecting the cells that pass through the first cell strainer, and
iii) sorting the cells collected in step ii) by fluorescent activated cell separation (FACS) based on forward scatter and collecting cells which are at least about 40µM in size.

In yet another embodiment of the above aspect, the method comprises
i) treating the sample to produce at least a partial single cell suspension, and
ii) sorting the at least partial single cell suspension by fluorescent activated cell separation (FACS) based on forward scatter and collecting cells which are between about 40µm and 100µm, in size.

In a further embodiment of the above aspect, the method further comprises positively and/or negatively selecting multinucleated fetal cells using a method as described above for the first aspect.

In another embodiment, following enrichment the multinucleated fetal cells are treated to produce a single nuclei suspension.

Also disclosed herein is an enriched population of fetal cells obtained by a method of the invention.

Furthermore, disclosed herein is a composition comprising fetal cells and a carrier.

Fetal cells processed and/or enriched using a method of the invention can be used to analyse the genotype of the fetus. Thus, in yet another aspect, the present invention provides a method for analysing the genotype of a fetal cell at a locus of interest, the method comprising
i) processing a transcervical sample using a method of the invention and/or enriching fetal cells using a method of the invention, and
ii) analysing the genotype of at least one fetal cell at a locus of interest.

The genotype of the fetus can be determined using any technique known in the art. Examples include, but are not limited to, karyotyping, hybridization based procedures, and/or amplification based procedures.

The genotype of a fetal cell can be analysed for any purpose. Typically, the genotype will be analysed to detect the likelihood that the offspring will possess a trait of interest. Preferably, the fetal cell is analysed for a genetic abnormality linked to a disease state, or predisposition thereto. In one embodiment, the genetic abnormality is in the structure and/or number or chromosomes. In another embodiment, the genetic abnormality encodes an abnormal protein. In another embodiment, the genetic abnormality results in decreased or increased expression levels of a gene.

In at least some instances, the enrichment methods of the invention will not result in a pure fetal cell population. In other words, some maternal cells may remain. Thus, in a preferred embodiment the methods of diagnosis (determination, analysis etc) further comprises identifying a cell as a fetal cell. This analysis may positively identify maternal or fetal cells. In the case of positively identifying maternal cells, the non-labelled cells will be fetal cells. Alternatively, both maternal and fetal cells are positively identified using different selectable markers, or a marker that results in a different level of signal between maternal and fetal cells. These procedures can be performed using any technique known in the art. For example, for male fetal cells a Y-chromosome specific probe can be used. In another example, telomere length is analysed. In a further embodiment, maternal cells are identified using an agent, such as an antibody, that binds a Class I MHC molecule. Other methods suitable to perform this embodiment are described herein.

The fetal cells can be used to determine the sex of the fetus. As a result, in a further aspect, the present invention provides a method of determining the sex of a fetus, the method comprising
i) processing a transcervical sample using a method of the invention and/or enriching fetal cells using a method of the invention, and
ii) analysing at least one fetal cell to determine the sex of the fetus.

The analysis of the fetal cells to determine the sex of the fetus can be performed using any technique known in the art. For example, Y-chromosome specific probes can be used, and/or the cells karyotyped.

The processed and/or enriched fetal cells can also be used to identify the father of the fetus. Accordingly, in a further aspect, the present invention provides a method of determining the father of a fetus, the method comprising
i) processing a transcervical sample using a method of the invention and/or enriching fetal cells using a method of the invention, and
ii) determining the genotype of the candidate father at one or more loci,
iii) determining the genotype of the fetus at one or more of said loci, and
iv) comparing the genotypes of ii) and iii) to determine the probability that the candidate father is the biological father of the fetus.

Whilst in some cases it may not be essential that the genotype of the mother also be analysed, for accuracy it is preferred that the method further comprises determining the genotype of the mother at one or more of said loci.

Analysis of the genotype of the candidate father, fetus or mother can be performed using any technique known in the art. One preferred technique is performing DNA fingerprinting analysis using probes/primers which hybridize to tandemly repeated regions of the genome. Another technique is to analyse the HLA/MHC region of the genome.

As will be apparent, preferred features and characteristics of one aspect of the invention are applicable to many other aspects of the invention.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The invention is hereinafter described by way of the following non-limiting Examples and with reference to the accompanying figures.

### BRIEF DESCRIPTION OF THE ACCOMPANYING DRAWINGS

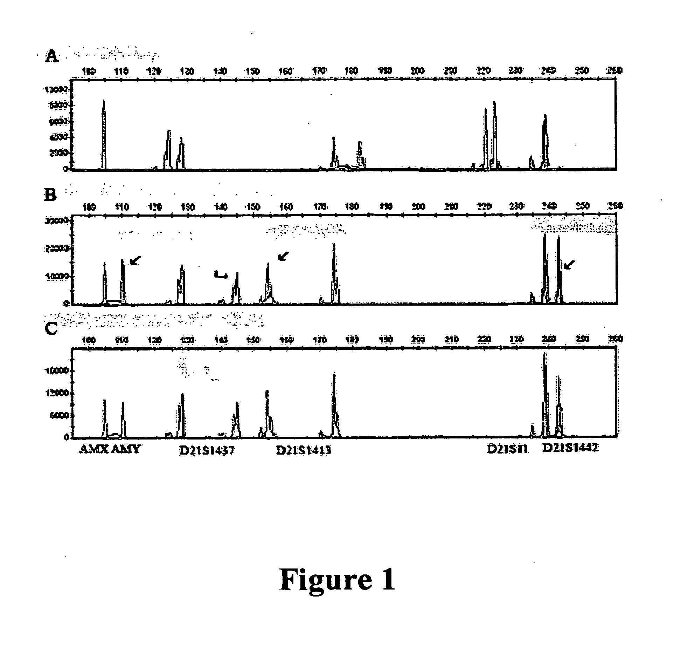

**Figure 1****.** Electrophoretogram of fluorescent amplified products of MACS sorted transcervical samples using positive and negative selection with chromosome 21 short tandem repeat (STR) markers and the sex marker, amelogenin. The x axis shows the calculated length of the polymerase chain reaction (PCR) products (in base pairs); the y axis shows fluorescent intensities in arbitrary units. Comparative analysis of the mother's STR profile (A) with the isolated fetal cells using either NDOG1 positive (B) or HLA negative (C) selection shows an identical paternally inherited STR profile confirming fetal origin. No maternal contamination was evident in either sample. The arrow indicates paternally inherited alleles at each locus.

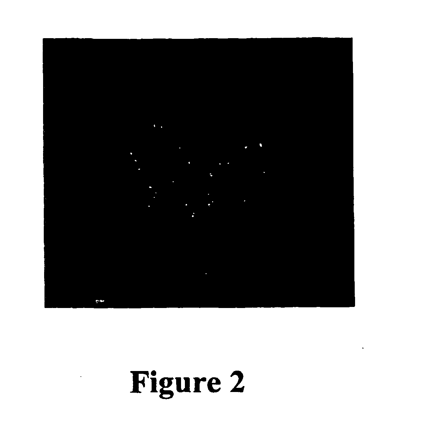

**Figure 2****.** Male multinucleated syncytiotrophoblasts in a NDOG1 positive sorted transcervical sample (positive selection) using FISH analysis. The sex chromosomes have been labelled with specific fluorescent probe red and green representing the X and Y-chromosomes.

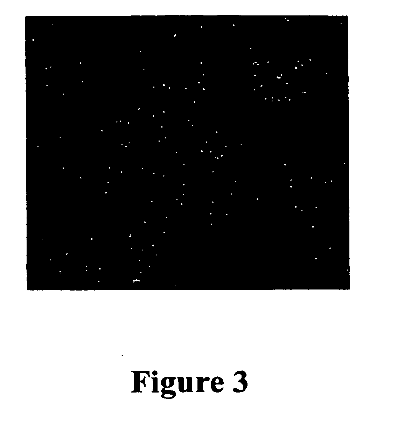

**Figure 3****.** Male fetal cells in a HLA sorted transcervical sample (negative selection) using FISH analysis. The sex chromosomes have been labelled with specific fluorescent probe red and green representing the X and Y-chromosomes.

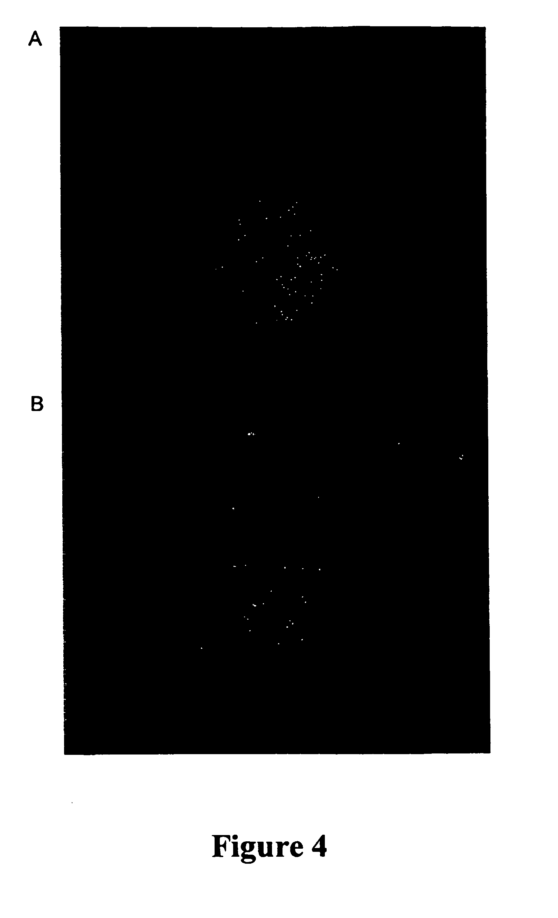

**Figure 4****.** Multinucleated syncytiotrophoblasts isolated using size selection (<100µm, >40µm in size). Fluorescent in-situ hybridisation (FISH) has been employed to label the sex chromosomes using fluorescent probes. The green and red dots represent the X and Y-chromosomes. (A) female fetus; (B) male fetus.

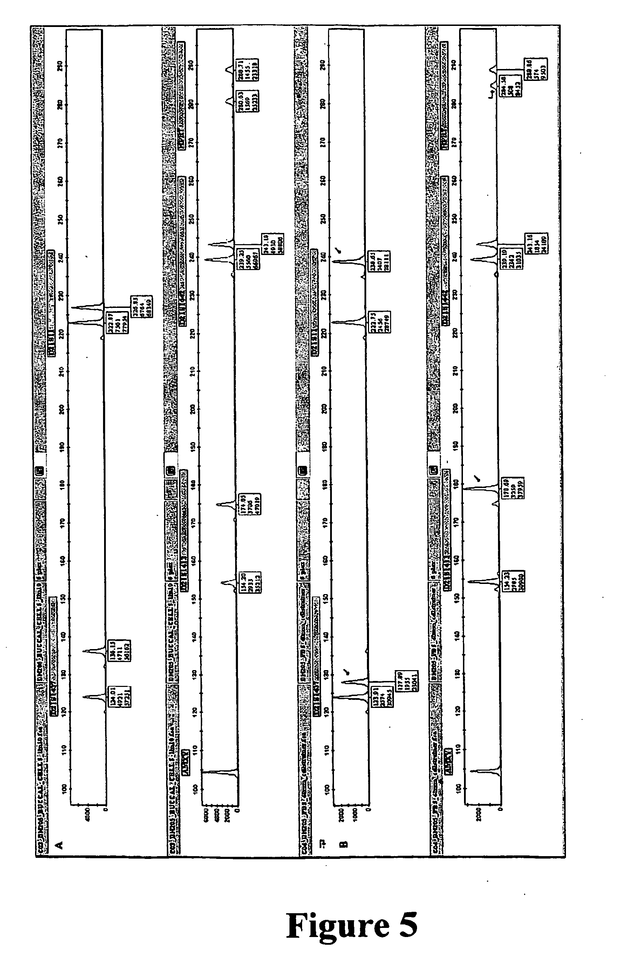

**Figure 5****.** Electrophoretogram of fluorescent amplified polymerase chain reaction (PCR) products following size selection with chromosome 21 short tandem repeat (STR) markers and the sex markers, amelogenin and polymorphic hypoxanthine guanine phosphoribosyl transferase (HPRT). The x-axis shows the calculated length of the amplified STR amplicons (in base pairs) and the y-axis shows fluorescent intensities in arbitrary units. Comparative analysis of the mother's STR profile (A) with the isolated fetal cells (<100µm, >40µm in size) STR profile (B) shows paternally inherited alleles confirming fetal origin of the isolated syncytiotrophoblasts. The example of fetal cells (B) shown is from a chromosome 21 disomic female fetus. The arrow indicates paternal inherited alleles for each locus.

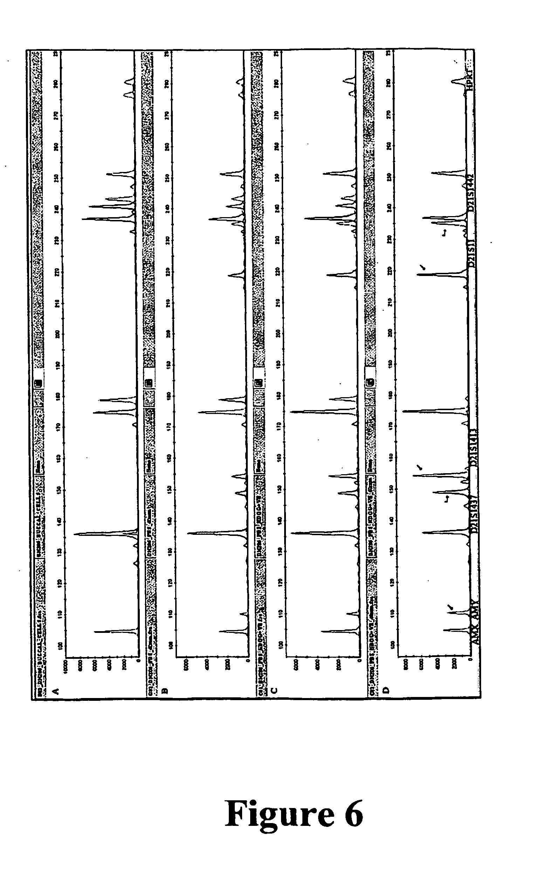

**Figure 6****.** Electrophoretogram of fluorescent amplified polymerase chain reaction (PCR) products of isolated syncytiotrophoblasts using 1 and 2 step enrichment process. The x-axis shows the calculated length of the amplified STR amplicons (in base pairs) and the y-axis shows fluorescent intensities in arbitrary units. Comparative analysis of the mother's STR profile (A) with the cells isolated by size selection (<100µm, >40µm in size) (B) and MACS using NDOG1 positive selection (C) showed a mixed DNA profile (fetal + maternal). The two-step process (D) improved the overall quality of the sample enabling a pure DNA profile ("clean fingerprint") to be obtained. The arrow indicates paternal inherited alleles for each locus.

### DETAILED DESCRIPTION OF THE INVENTION

### General Techniques and Abbreviations

Unless specifically defined otherwise, all technical and scientific terms used herein shall be taken to have the same meaning as commonly understood by one of ordinary skill in the art (e.g., in cell culture, fetal cell biology, molecular genetics, immunology, immunohistochemistry, protein chemistry, nucleic acid hybridization, flow cytometry, and biochemistry).

Unless otherwise indicated, the recombinant protein, cell culture, and immunological techniques utilized in the present invention are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J.E. Coligan et al. (editors) Current Protocols in Immunology, John Wiley & Sons (including all updates until present).

### Abbreviations used herein include;

BSA: Bovine Serum Albumin
DAPI: Diamidino-2-phenylindonle
EDTA: Ethylenediaminetetra-acetic Acid
FACS: Fluorescence Activated Cell Sorting
FISH: Fluorescent In Situ Hybridization
HLA: Human Leukocyte Antigen
MACS: Magnetic Activated Cell Sorting
MHC: Major Histocompatibility Complex
PBS: Phosphate Buffered Saline
QFPCR: Quantitative Fluorescent Polymerase Chain Reaction
STR: Short Tandem Repeat
TCC: Trans Cervical Cell.

### Sample, Processing and/or Enrichment of Cells

As used herein, the term "transcervical sample" refers to material taken directly from the pregnant female comprising cervical mucous, as well as such material that has already been partially purified. Examples of such partial purification include the removal of at least some non-cellular material, removal of maternal red blood cells, and/or removal of maternal lymphocytes. In some embodiments, the cells in the sample are cultured *in vitro* before a method of the invention is performed. When the sample is to be used for a method of processing of the invention it is preferred that it has not been subjected to any partial purification procedures.

In an embodiment, red blood cells are removed from the sample. Red blood cells can be removed using any technique known in the art. Red blood cells (erythrocytes) may be depleted by, for example, density gradient centrifugation over Percoll, Ficoll, or other suitable gradients. Red blood cells may also be depleted by selective lysis using commercially available lysing solutions (eg, FACSlyse^{™}, Becton Dickinson), Ammonium Chloride based lysing solutions or other osmotic lysing agents.

The transcervical sample can be obtained using a variety of sampling methods including, but not limited to, aspiration, irrigation, lavage and cell extraction. The sample may be obtained from sites including, but not limited to, the endocervical canal, external os, internal os, lower uterine pole and uterine cavity. A range of devices are available commercially which may be suitable for obtaining the sample, including but not limited to: "Aspiracath" aspiration catheter (Cook Medical, IN, USA), "Tao" brush endometrial sampler (Cook Medical, IN, USA), Goldstein Sonobiopsy catheter (Cook Medical,IN, USA), Aspiration kit (MedGyn, IL, USA), Endosampler (MedGyn, IL, USA), Endometrial sampler and cervical mucus sampling syringe (Rocket Medical, UK), "Sampling Probet" (Gyn 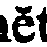 tics Products, Belgium), "Sampling in-out" - endometrial curette (Gynetics Products, Belgium), Endometrial cell sampler (Cheshire Medical Specialities Inc, CT, USA), Aspirette® Endocervical Aspirator and Embryo Transfer Catheter (Cooper Surgical, CT, USA), and Intrauterine Catheter (Cooper Surgical, CT, USA).

In a preferred embodiment, the transcervical sample is/was obtained from the endocervical canal.

In a preferred embodiment, the transcervical sample is/was obtained using a flexible aspiration catheter, uterine lavage, a cytobrush or an endocervical lavage, more preferably a flexible aspiration catheter.

In a preferred embodiment, the transcervical sample is/was obtained without the use of internal imaging such as ultrasound.

Once obtained, the sample is preferably stored at 0 to 4°C until use to minimize the number of dead cells, cell debris and cell clumps. The sample is preferably transported and/or stored in HypoThermosol- FRS (HTS-FRS) Medium (Biolife Solutions) at 4°C. For long term storage, the sample is preferably stored in CryoStor CS5(Biolife Solutions) at -80°C.

In a further embodiment, the sample is transported and/or stored in Gibco^{™} AmnioMaxII, Gibco^{™} AmnioMax C-100, or Gibco^{™} Keratinocyte-SFM supplemented with 2% fetal bovine serum, heparin (2500U), hydrocortisone (5 µg/ml), insulin (5 µg/ml), human epidermal growth factor (5µg/ml), human basic fibroblast growth factor (5µg/ml), 25 µg/ml gentamycin, 50 ng/ml amphotericin B, 1-2 mmol/L vitamin C (ascorbic acid) or a water soluble analogue of vitamin E (1mmol/L Trolox).

In one embodiment, the transport and/or storage media comprises serum such as bovine calf serum or human serum.

In a further embodiment, the storage medium is degassed with Nitrogen to reduce oxidative stress to the samples.

In a preferred embodiment, the multinucleated fetal cells are syncytiotrophoblasts. "Syncytiotrophoblasts" are found in the placenta of human embryos. They are the outer syncytial layer of the trophoblasts and actively invade the uterine wall. They form the outermost fetal component of the placenta (also known as 'syntrophoblast') and massively increase the surface area available for nutrient exchange between the mother and the fetus.

"Cytotrophoblasts" form the inner layer of the trophoblasts, interior to the syncytiotrophoblast in an embryo. They serve to anchor the embryonic chorion to the maternal endometrium. Cytotrophoblasts are stem cells in the chorionic villi. During differentiation, mononuclear cytotrophoblast fuse together into the multinucleated syncytiotrophoblasts.

As used herein, the terms "enriching", "enrichment" and "enriched" are used in their broadest sense to encompass the isolation of the fetal cells such that the relative concentration of fetal cells to non-fetal cells in the treated sample is greater than a comparable starting material. Preferably, the enriched fetal cells are separated from at least 10%, more preferably at least 20%, more preferably at least 30%, more preferably at least 40%, more preferably at least 50%, more preferably at least 60%, more preferably at least 70%, more preferably at least 75%, more preferably at least 80%, more preferably at least 90%, more preferably at least 95%, and even more preferably at least 99% of the non-fetal cells in the sample obtained from the mother. In one embodiment, the enriched cell population contains no maternal cells (namely, pure). The terms "enrich" and variations thereof are used interchangeably herein with the term "isolate" and variations thereof. Furthermore, a population of cells enriched using a method of the invention may only comprise a single fetal cell. In addition, the enrichment methods of the invention may be used to isolate a single fetal cell.

As used herein, the term "treating the sample to produce at least a partial single cell suspension" refers to any procedure that disaggregates cells that are in clumps whilst not disrupting their integrity, however, it will be appreciated by the skilled person that within a population of cells this step may disrupt the integrity of at least some cells. Furthermore, as the skilled addressee will appreciate there may be at least some contaminating aggregated cells following conducting this step. In a particularly preferred embodiment, this step is achieved by at least partially mechanically disaggregating the sample. In alternate embodiment, this step is achieved by at least partially enzymatically disaggregating the sample.

As used herein, the term "at least partially" means that the relevant step increases the number of, and/or the cell suspension comprises, single cells not aggregated to other cells (directly or indirectly). In a preferred embodiment, at least 50%, more preferably at least 70%, more preferably at least 80%, more preferably at least 90%, more preferably at least 95%, more preferably at least 97%, more preferably at least 99%, and even more preferably 100% of the cells are single cells following the relevant step.

As used herein, the term "at least partially mechanically disaggregating the sample" refers to using non-chemical or non-enzymatic means to disassociate at least some aggregated cells following removal of the sample from the pregnant female. As the skilled addressee will appreciate, this step must not be result in the destruction of a significant number of cells. Preferably, at least 50%, more preferably at least 60%, more preferably at least 70%, more preferably at least 80%, more preferably at least 90%, more preferably at least 95%, and even more preferably 100% of the cells in the sample have not been destroyed following this step. Examples of methods for mechanically disaggregating the sample include, but are not limited to, gentle pipetting using an about 1ml pipette, using forceps, fluid agitation, fluidics movement and/or cutting. Examples of fluid agitation include, but are not limited to, spinning in a vortex, centrifuge or suspension mixer; shaking in a water bath; and stirring using a magnetic stirrer. The fluid agitation should create enough shear force to partially disaggregate the sample. Examples of fluid movement are using pressure or vacuum to disperse cells by passing the fluid through channels/tube/orifice. In a preferred embodiment, mechanically disaggregating the sample comprises gentle pipetting using an about 1 ml pipette and/or using forceps. In one example, the sample it pipetted using a 1 ml pipette until it can easily go up and down the tip.

As used herein, the term "selecting cells based on cell size" refers to the isolation of single cells based on their size. Preferably, at least 70%, more preferably at least 80%, more preferably at least 90%, more preferably at least 95%, more preferably at least 97%, more preferably at least 99%, and even more preferably 100% of the cells obtained in the relevant step are single cells.

As used herein, the term "cell size" refers to the dimensions of the cell. Often, the fetal cells will be spherical, and hence cell size refers to the diameter of the cell. However, in some instances at least some of the fetal cells may be non-spherical. For non-spherical cells, enrichment is based on the smallest diameter of the cell, for example, such that they are able to be selected using a cell strainer with a mesh size as defined herein in instances where the cells are to pass through the cell strainer.

The step of "selecting cells based on cell size" can be performed using any procedure known in the art. In a preferred embodiment, this step comprises passing the sample through a suitable cell strainer.

As used herein, the term "enzyme capable of disassociating aggregated cells" refers to any protein which is able degrade material between the aggregated cells. Examples include, but are not limited to, contacting the sample with a collagenase, a protease or a combination thereof.

Proteases (or proteinases) hydrolyze the protein portions of the sample. In one example, an enzyme cocktail, such as pronase which cleaves almost any peptide bond, is used to digest extracellular proteins in a sample. Pronase includes both endo-proteinases and exo-proteinases. Numerous proteolytic compounds that are useful for hydrolyzing proteins are known in the art. Many of these compounds, such as trypsin, chymotrypsin, pepsin, and papain, may be used in addition to or *in lieu* of pronase.

Commercially available mixes of enzymes for treating the non-single cell material include, but are not limited to, liberase blendzyme which is a combination of collagenase isoform, and thermolysin which can be obtained from Roche.

In an embodiment, the sample or "material which did not pass through the cell strainer" is treated with a mucolytic agent prior to, or in combination with, being treated with the enzyme to disassociate the cells. Suitable mucolytic agents may be selected from the group including N-acetyl-L-cysteine, DTT, trypsin and trypsin/EDTA. Preferably, the mucolytic agent is N- acetyl-L-cysteine.

The methods of the invention can be performed on any pregnant female of any mammalian species. Preferred mammals include, but are not limited to, humans, livestock animals such as sheep, cattle and horses, as well as companion animals such as cats and dogs..

The sample may be obtained at any stage of pregnancy. Preferably the sample is obtained during the first or second trimester of pregnancy. More preferably, the sample is obtained in the first trimester of pregnancy. Preferably the sample is obtained up to 14 weeks of the pregnancy. Even more preferably, the transcervical sample was/is obtained within 5 to 12 weeks of pregnancy. Ideally the sample is obtained at a stage when a decision can be made for the well-being of the fetus and preferably within a period where an opportunity to make an early decision regarding therapeutic abortion can be made.

In an embodiment, the methods of the invention further comprise obtaining the transcervical sample.

### Using Cell Size to Process and/or Enrich Fetal Cells

Any method known in the art which can be used to select and/or enrich cells based on cell size can be used in the methods of the invention. Examples include, but are not limited to, cell strainers, flow cytometry and/or microfluidics, or a combination thereof.

In one aspect, the present invention comprises the step of "selecting cells based on cell size". For example, a cell strainer with a mesh size of about 100µM can be used such as the 100 µm Nylon Strainer sold by Becton Dickinson, USA. As another example, large cell columns for magnetic sorting made by Miltenyi Biotec GmBH, Germany, which have mesh sizes of 50-100µM could be used. In a preferred embodiment, the strainer has a mesh size of about 100µM. More specifically, the mesh size should be sufficiently large to allow trophoblasts such as Syncytiotrophoblasts to pass through the column, whilst being sufficiently small to not allow clumps comprising two or more, more preferably five or more, cells to pass through the column.

Another aspect of the invention specifically relates to the enrichment of multinucleated fetal cells, however, following enrichment these cells could be combined with other, non-multinucleated fetal cells. In a preferred embodiment, this aspect uses one or more cell strainers which can be made from, for instance, nylon or metal. In an embodiment, a single strainer can be used which comprises two different mesh sizes. For example, the apparatus can comprise a top mesh which has a large pore (mesh) size (for example 100µM) and a bottom mesh which has a smaller pore (mesh) size than the top mesh (for example 40µM). The cell suspension or sample is placed on the top mesh, and cells which pass through the top mesh but do hot pass through the bottom mesh are collected. The method comprises selecting cells which are between about 40µm and 150µm in size, about 40µm and 100µm in size, or between about 40µm and 70µm in size.

Cell strainers can be made from any suitable material, for instance, nylon or metal. For example, a nylon cell strainer(s) with a mesh size of about 100µm, about 70µm and/or about 40µm can be used such as those sold by Becton Dickinson USA, BD Biosciences, Stem Cell Technologies and Miltenyi Biotech.

In flow cytometry, a beam of laser light is projected through a liquid stream that contains cells, or other particles, which when struck by the focussed light give out signals which are picked up by detectors. These signals are then converted for computer storage and data analysis, and can provide information about various cellular properties. In some embodiments of the present invention, forward scatter data can be used to select and/or enrich fetal cells, either multinucleated and/or non-multinucleated, based on cell size. For example, when a laser hits the cell, the larger the cell the more photons of light it scatters. By measuring the light scattered on the side of a cell furthest from where the laser hits the cell, a measure of cell size can be obtained.

Many larger flow cytometers are also "cell sorters", such as fluorescence-activated cell sorters (FACS), and are instruments which have the ability to selectively deposit cells from particular populations into tubes, or other collection vessels. In an embodiment, the cells are isolated using FACS. This procedure is well known in the art and described by, for example, Melamed, et al. Flow Cytometry and Sorting Wiley-Liss, Inc., New York, N.Y. (1990); Shapiro Practical Flow Cytometry, 4 ed, Wiley-Liss, Hoboken, NJ. (2003); and Robinson et al. Handbook of Flow Cytometry Methods Wiley-Liss, New York, N.Y (1993); Harkins and Galbraith (1987) and US 4,765,737.

In order to sort cells, the instruments electronics interprets the signals collected for each cell as it is interrogated by the laser beam and compares the signal with sorting criteria set on the computer. If the cell meets the required criteria, an electrical charge is applied to the liquid stream which is being accurately broken into droplets containing the cells. This charge is applied to the stream at the precise moment the cell of interest is about to break off from the stream, then removed when the charged droplet has broken from the stream. As the droplets fall, they pass between two metal plates, which are strongly positively or negatively charged. Charged droplets get drawn towards the metal plate of the opposite polarity, and deposited in the collection vessel, or onto a microscope slide, for further examination.

The cells can automatically be deposited in collection vessels as single cells or as a plurality of cells, e.g. using a laser, e.g. an argon laser (488 nm) and for example with a Flow Cytometer fitted with an Autoclone unit (Coulter EPICS Altra, Beckman-Coulter, Miami, Fla., USA). Other examples of suitable FACS machines useful for the methods of the invention include, but are not limited to, MoFlo^{™} High-speed cell sorter (Dako-Cytomation Ltd), FACS Aria^{™} (Becton Dickinson), ALTRA^{™} Hyper sort (Beckman Coulter) and CyFlow^{™} sorting system (Partec GmbH).

As noted above, microfluidics can also be used to select and/or enrich fetal cells using the methods of the invention. A microfluidic device can be identified by the fact that it has one or more channels with at least one dimension less than 1 mm. Common fluids used in microfluidic devices include whole blood samples, bacterial cell suspensions, protein or antibody solutions and various buffers. The use of microfluidic devices to conduct biomedical research and create clinically useful technologies has a number of significant advantages. First, because the volume of fluids within these channels is very small, usually several nanoliters, the amount of reagents and analytes used is quite small. The fabrication techniques used to construct microfluidic devices, discussed in more depth later, are relatively inexpensive and are very amenable both to highly elaborate, multiplexed devices and also to mass production. Furthermore, microfluidic technologies enable the fabrication of highly integrated devices for performing several different functions on the same substrate chip. Examples of the use of microfluidics to select and/or enrich cells based on size are described in WO 2004/113877, Murthy *et al.* (2006), Wu *et al.* (2007) and Inglis *et al.* (2008). Considering the present disclosure, the same procedures can readily be adapted by those skilled in microfluidics to select and/or enrich fetal cells.

In a preferred embodiment, the sample/cells are not centrifuged at least until after the cells are selected and/or enriched based on cell size.

Following processing and/or enrichment, the resulting cells can be cultured *in vitro* to expand fetal cells numbers using techniques known in the art. For example, culturing in RPMI 1640 media (Gibco).

### Negatively Selecting Fetal Cells

As the skilled person will appreciate, negatively selecting fetal cells comprises removing from the sample cells that are identified/labelled as maternal. In other words, maternal cells are positively selected from the sample by targeting a molecule preferentially expressed in the maternal cells but not expressed in at least some fetal cells.

### Major Histocompatibility Complex

The major histocompatibility complex (MHC) includes at least three classes of genes. Class I and II genes encode antigens expressed on the cell surface, whilst class III genes encode several components of the complement system. Classes I and II antigens are glycoproteins that present peptides to T lymphocytes. Human MHC molecules are also known in the art as Human Leukocyte Antigens (HLA). Thus, the terms "HLA" and "MHC" are often used interchangeably herein.

Human and murine class I molecules are heterodimers, consisting of a heavy alpha chain (45kD) and a light chain, beta-2-globulin (12kD). Class I molecules are found on most, if not all, nucleated cells. The alpha chain can be divided into three extracellular domains, alpha1, alpha2 and alpha3, in addition to the transmembranous and cytoplasmic domains. The alpha3 domain is highly conserved, as is beta-2-microglobulin. Both alpha3 domain and beta-2-microglobulin are homologous to the CH3 domain of human immunoglobulin.

Class II molecules are heterodimeric glycoproteins, alpha chain (34kD) and beta chain (29kD). Each chain has 2 extracellular domains, together with the transmembranous and cytoplasmic domains. The membrane-proximal alpha2 and beta2 domains are homologous to immunoglobulin CH domain. Class II molecules are less commonly expressed when compared to Class I, typically being found in dendritic cells, B lymphocytes, macrophages, and a few other cell types.

There are 3 class I loci (B,C,A) in the short arm of human chromosome 6, and 4 loci (K, D(L), Qa, Tla) in murine chromosome 17. These loci are highly polymorphic. The variable residues are clustered in 7 subsequences, 3 in alpha1 domain and 4 in alpha2 domain. There are 3 major human class II loci (HLA-DR, HLA-DO, HLA-DP) and 2 murine loci (H-2I-A, H-2I-E). All class II beta chains are polymorphic. Human HLA-DQ alpha chain is also polymorphic.

Preferably, at least some methods of the invention utilize an agent (preferably an antibody) which binds at least one MHC molecule. Preferably, the agent binds an extracellular portion of the MHC molecule. This has at least two advantages, i) the method of the invention can be used to enrich live fetal cells, and ii) an additional step of ensuring that the agent passes through the cell membrane (for example having to fix and permeabilize the cell) is not required.

Preferably, the agent is capable of binding at least one Class I HLA molecule. In one embodiment, the agent is capable of binding HLA-A, HLA-B and HLA-C molecules. In a preferred embodiment, the agent is capable of binding HLA-A and/or HLA-B molecules. In a further embodiment, at least two different agents can be used that bind the same or different Classes or sub-classes of MHC molecules.

As used herein, a "sub-class" of a MHC molecule is a distinct type of MHC molecules of a particular Class. For example, HLA-A molecules and HLA-B molecules are each considered herein as a sub-class of Class I MHC molecules.

In a preferred embodiment, the method comprises
i) contacting the cells with an agent that binds at least one MHC molecule, and
ii) removing cells bound by the agent.

As the skilled addressee will appreciate more than one agent which binds an MHC molecule can be used. For example, in an embodiment, the method comprises contacting the cells with i) an agent that binds at least one Class I MHC molecule, and ii) an agent that binds at least one Class II MHC molecule.

In another embodiment, the agent binds:
i) a monomorphic determinant of HLA-A molecules,
ii) a monomorphic determinant of HLA-B molecules, or
iii) a monomorphic determinant of HLA-A and HLA-B molecules.

As used herein, a "monomorphic determinant" refers to a region of a group proteins that is highly conserved between at least 90%, more preferably at least 95%, more preferably at least 99%, and even more preferably 100% of the group which can be recognised by a suitable binding agent such as an antibody. The region can be a continuous stretch of amino acids, and/or a group of highly conserved amino acids that, upon protein folding, are closely associated. For example, a "monomorphic determinant" of a Class I MHC molecule is a region of the proteins (isotypes) encoded by different alleles of Class I MHC genes that is highly conserved between the different proteins of the Class and that can be bound by the same antibody.

In one embodiment, the agent does not bind HLA-C.

In an alternate embodiment, the agent binds a monomorphic determinant of HLA-A, HLA-B and HLA-C molecules. In this embodiment it is preferred that the agent is used at sub-saturating concentrations.

In yet a further embodiment, more than two agents are used which bind different alleles of the same class of MHC molecule. Preferably, collectively the agents bind all alleles of the same class of MHC molecule.

Compounds have been shown to associate *in situ* with MHC molecules, and hence these compounds can be targeted using the methods of the invention. Accordingly, in another embodiment, the method comprises
i) contacting cells in the sample with an agent that binds a compound that associates with an MHC molecule, and
ii) removing cells bound by the agent.

For example, the compound could be a ligand, for example a protein ligand, that binds an MHC molecule.

In an embodiment, the genotype of an MHC allele is not determined for the mother, father and/or fetus.

### Other Maternal Cell Specific Markers

The essential feature for choosing other maternal cell specific markers is that they are not expressed on at least the majority of fetal cells. Those skilled in the art are aware that the types of nucleated maternal cells in maternal blood include B cells, T cells, monocytes, macrophages dendritic cells and stem cells, each characterised by a specific set of surface markers that can be targeted for depletion. Examples of non-MHC molecules which can be targeted to possibly further deplete the sample of maternal cells include, but are not limited to, CD3, CD4, CD8, CD10, CD14, CD15, CD45, CD56 and proteins described by Blaschitz *et al.* (2000). Such further maternal cell specific agents can readily be used in combination with an agent that binds at least one MHC molecule. For example, magnetic beads can be produced which have both anti-MHC and anti-CD45 antibodies attached thereto.

Further examples of maternal cells that may be depleted include, but are not limited to, vaginal epithelial cells, cervical epithelial cells, endometrial cells, maternal endothelial cells, maternal placental cells, polymorphs and mesenchymal cells of the placental villi.

### Positively Selecting Fetal Cells

Fetal cell can be positively selected by using agents which bind molecules, typically proteins, which are not significantly produced by maternal cells in the sample. Examples of fetal cell markers include, but are not limited to, any molecule which is expressed by syncytiotrophoblasts and/or cytotrophoblasts but is not expressed by maternal cells. Examples include, but are not limited to, NDOG1 (AbCam, GeneTex, Serotec), NDOG2, Human Chorionic Gonadotropin (Calbiochem), MCP/cd46 (trophoblast/lymphocyte cross-reactive protein) (Abnova), TPBG (Trophoblast glycoprotein) (Abnova), GCSF receptor, ADFP (Adipose Differentiation Related Protein) (GenWay), Apolipoprotein H (AbCam), Placental Alkaline Phosphatase (AbCam), CXCR6 (Chemokine receptor 6) (R&D Systems), HLA-G (AbCam), CHL1 (extravillous cytotrophoblast antigen) (Abnova), Cytokeratin 7 (AbCam), Cytokeratin 8 (AbCam), Cytokeratin 18 (AbCam), FAS-Associated Phosphatase-1 (Leica), Folate Binding Protein (AbCam), FD0161G, Glucose Transporter GLUT3, H315, H316, HAI-1 (Hepatocyte growth factor activator protein-1 (EBioscience)), Human Placental Lactogen (Serotec), Id-1, Id-2, IBSP (Integrin Binding SialoProtein), MCSF-Receptor, MNF116, OKT9, plasminogen activator inhibitor 1 (AbCam), PLP-A (prolactin like proteins A) (Millipore Corporation), PLP-B (prolactin like proteins B), PLP-C (prolactin like proteins C), PLP-D (prolactin like proteins D), PLP-F (prolactin like proteins F), PLP-L (prolactin like proteins L), PLP-M (prolactin like proteins M), PLP-N (prolactin like proteins N), SP-1 (trophoblast specific beta 1 glycoprotein) (AbCam, BD Pharmingen), SSEA (Stage Specific Embryonic Antigen) (Novus Biologicals), TA1, TA2, Tfeb, Tromal, Trop1 (EBioscience) and Trop2, URO-4 (Adenosine Deaminase Binding Protein [ABP]) (Covance), or combination of any two or more thereof.

In a particularly preferred embodiment, the fetal cells are positively selected using an agent which binds syncytiotrophoblasts such as a monoclonal antibody which binds NDOG 1.

In a further preferred embodiment, the fetal cells are positively selected using combinations of agents which bind to villous syncytiotrophoblasts, villous cytotrophoblasts and extra villous cytotrophoblasts. For example, the combination of agents may include an agent which binds NDOG1 (Syncytiotrophoblasts), an agent which binds SP-1 (Villous Cytotrophoblasts and villous syncytiotrophoblasts), and an agent which binds HLA-G (ExtraVillous Cytotrophoblasts).

### Telomeres and Telomerases

Telomeres consist of DNA-protein complexes that are located at the ends of eukaryotic chromosomes and function to provide protection against genome instability promoting events such as degradation of the terminal regions of chromosomes, fusion of a telomere with another telomere or broken DNA end, or inappropriate recombination. Telomeres prior to birth can be considered to be at maximum length. After birth, with each cell division, they get progressively shorter (Vaziri *et al.,* 1994). Telomeric DNA comprises tandem repeats of DNA, in humans the 6-base pair sequence TTAGGG, that form a molecular scaffold containing binding sites for telomeric proteins, resulting in a dynamic DNA-protein complex at the telomere.

Telomerase is an enzyme concerned with the formation, maintenance, and renovation of telomeres at the ends of chromosomes. Telomerase acts as an RNA-dependent DNA polymerase that synthesizes telomeric DNA sequences and consists of two essential components; the first being the functional RNA component (in humans also known as hTR) and the other being the catalytic protein (in humans also known as hTERT). Hence, telomerase is a ribonucleoprotein. Telomerase regulates the proliferative capacity of cells. Telomerase is now classed as a tumour-associated antigen. It may also play a role in the clonal expansion of lymphocytes in response to viral infection.

In biochemical terms, telomerase acts as a telomerase reverse transcriptase (TERT). It transcribes RNA into DNA and is the reverse-transcribing enzyme specific to the telomeric sequence. It has two unique features: it is able to recognize a single-stranded (G-rich) telomere primer and it is able to add multiple telomeric repeats to its end by using its RNA moiety as a template.

The correlation between telomerase activity, telomere lengths, and cellular replicative capacity has led to the theory that maintenance of telomere lengths by telomerase acts as a molecular clock to control replicative capacity and senescence.

The RNA components of human and other telomerases have been cloned and characterized (WO 96/01835). However, the characterization of all the protein components of telomerase has been difficult. Despite this, a number of proteins that may interact with TERT have been identified and include TEP-1 (telomerase associated protein 1) (Harrington *et al.,* 1997) and 14-3-3 proteins (Seimiya *et al.,* 2000).

As used herein, the term "telomerase" refers to at the least the ribonucleoprotein comprising the functional RNA component and the reverse transcriptase. However, at least in some instances this term may also encompass other proteins which may form part of the telomerase complex such as the TEP-1 and 14-3-3 proteins.

Telomerases and telomeres can be used in the positive selection of fetal cells using the procedures outlined in WO 2006/119569.

### Agent

The present invention relies on the use of various agents which bind molecules expressed by maternal or fetal cells. These agents can be of any structure or composition as long as they are capable binding to a target molecule. In one embodiment, the agents useful for the present invention are proteins. Preferably, the protein is an antibody or fragment thereof.

Antibodies or fragments thereof useful for the methods of the invention can be, but are not limited to,
- a monoclonal antibody,
- a polyclonal antibody,
- Fab fragment which contains a monovalent antigen-binding fragment of an antibody molecule that can be produced by digestion of whole antibody with the enzyme papain to yield an intact light chain and a portion of one heavy chain;
- Fab' fragment which can be obtained by treating whole antibody with pepsin, followed by reduction, to yield an intact light chain and a portion of the heavy chain; two Fab' fragments are obtained per antibody molecule;
- (Fab')₂ fragment which can be obtained by treating whole antibody with the enzyme pepsin without subsequent reduction; F(ab)2 is a dimer of two Fab' fragments held together by two disulfide bonds;
- Fv, defined as a genetically engineered fragment containing the variable region of the light chain and the variable region of the heavy chain expressed as two chains;
- single chain antibody ("SCA"), defined as a genetically engineered molecule containing the variable region of the light chain, the variable region of the heavy chain, linked by a suitable polypeptide linker as a genetically fused single chain molecule; such single chain antibodies may be in the form of multimers such as diabodies, triabodies, and tetrabodies etc which may or may not be polyspecific (see, for example, WO 94/07921 and WO 98/44001) and
- single domain antibody, typically a variable heavy domain devoid of a light chain.

Furthermore, the antibodies and fragments thereof may be humanised antibodies, for example as described in EP 239400.

Antibodies useful for the methods of the invention can readily be produced using techniques known in the art. Alternatively, at least some suitable antibodies can be obtained from commercial sources. Anti-MHC antibodies can be obtained from commercial sources such as US Biological (Massachusetts, USA) and Chemicon International Inc. (California, USA). Furthermore, at least some anti-telomerase antibodies can be obtained from commercial sources such as Abcam Ltd (Cambridge, UK) and Calbiochem (California, USA).

The term "binds specifically" refers to the ability of the antibody to bind to a target ligand (such as an MHC molecule) but not significantly to other proteins in the sample.

If polyclonal antibodies are desired, a selected mammal (e.g., mouse, rabbit, goat, horse, etc.) is immunised with a suitable immunogenic polypeptide. Serum from the immunised animal is collected and treated according to known procedures. If serum containing polyclonal antibodies contains antibodies to other antigens, the polyclonal antibodies can be purified by immunoaffinity chromatography. Techniques for producing and processing polyclonal antisera are known in the art.

Monoclonal antibodies can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced can be screened for various properties; i.e., for isotype and epitope affinity.

An alternative technique involves screening phage display libraries where, for example the phage express single chain antibody (scFv) fragments on the surface of their coat with a large variety of complementarity determining regions (CDRs). This technique is well known in the art.

The binding of the agent to a maternal cell can be detected directly or indirectly. Direct detection relies on the agent being bound to a detectable label or isolatable label. Indirect detection relies on binding to the agent a detectable label or isolatable label, for example a detectably labelled secondary antibody, which binds the agent/maternal cell complex. Preferably, the label is selected from, but not limited to, the group consisting of: a fluorescent label, a radioactive label, a paramagnetic particle, a chemiluminescent label, a label that is detectable by virtue of a secondary enzymatic reaction, and a label that is detectable by virtue of binding to a molecule. In a particularly preferred embodiment, the detectable label or isolatable label is a paramagnetic particle.

The terms "detectable" and "isolatable" label are generally used herein interchangeably. Some labels useful for the methods of the invention cannot readily be visualized (detectable) but nonetheless can be used to enrich (isolate) fetal cells (for example a paramagnetic particle).

Exemplary labels that allow for direct measurement of antibody binding include radiolabels, fluorophores, dyes, magnetic beads, chemiluminescers, colloidal particles, and the like. Examples of labels which permit indirect measurement of binding include enzymes where the substrate may provide for a coloured or fluorescent product. Additional exemplary labels include covalently bound enzymes capable of providing a detectable product signal after addition of suitable substrate. Examples of suitable enzymes for use in conjugates include horseradish peroxidase, alkaline phosphatase, malate dehydrogenase and the like. Where not commercially available, such antibody-enzyme conjugates are readily produced by techniques known to those skilled in the art. Further exemplary detectable labels include biotin, which binds with high affinity to avidin or streptavidin; fluorochromes (e.g., phycobiliproteins, phycoerythrin and allophycocyanins; fluorescein and Texas red), which can be used with a fluorescence activated cell sorter; haptens; and the like.

Examples of fluorophores which can be used to label antibodies includes, but are not limited to, Fluorescein Isothiocyanate (FITC), Tetramethyl Rhodamine Isothiocyanate (TRITC), R-Phycoerythrin (R-PE), Alexa^{™}, Dyes, Pacific Blue^{™}, Allophycocyanin (APC), and PerCP^{™}.

The label may also be a quantum dot. In the context of antibody labelling they are used in exactly the same way as fluorescent dyes. Quantum Dots are developed and marketed by several companies, including, Quantum Dot Corporation (USA) and Evident Technologies (USA). Examples of antibodies labelled with quantum dots are described in Michalet *et al.* (2005) and Tokumasu and Dvorak (2003).

As noted above, in some embodiments the agent is not directly labelled. In this instance, cells are identified using another factor, typically a detectably labelled secondary antibody. The use of detectably labelled secondary antibodies in methods of detecting a marker of interest are well known in the art. For example, if an antibody was produced from a rabbit, the secondary antibody could be an anti-rabbit antibody produced from a mouse.

As used herein, the term "sub-saturating concentrations" of an agent such as an antibody means that the number of molecules of the agent is less, preferably significantly less, than the number of target molecules (for example MHC Class I molecules) in a sample. Thus, in this situation only a small fraction of target antigens per cell get an agent bound to them. For example, in some embodiments the ratio of agent to target is less than 1:10, 1:100, 1:1000, or 1:10000. Sub-saturating concentrations of an agent can readily be determined by the skilled person using standard techniques.

Maternal cells bound by an antibody can be killed, and thus depleted from a sample, by complement-dependent lysis. For example, antibody labelled cells can be incubated with rabbit complement at 37°C for 2 hr. Commercial sources for suitable complement systems include Calbiochem, Equitech-Bio and Pel Freez Biologicals. Suitable anti-MHC antibodies for use in complement-dependent lysis are known in the art, for example the W6/32 antibody (AbCam).

### Labelled Fetal Cell Detection and Isolation

Fetal cells can be positively and/or negatively selected using a variety of techniques well known in the art, including cell sorting, especially fluorescence-activated cell sorting (FACS), by using an affinity reagent bound to a substrate (e.g., a plastic surface, as in panning), or by using an affinity reagent bound to a solid phase particle which can be isolated on the basis of the properties of the solid phase particles for example beads (e.g., coloured latex beads or magnetic particles). Naturally, the procedure used will depend on whether maternal or fetal cells are being selected and how the cells have been labelled.

For selection of cells by cell sorting, the cells are labelled directly or indirectly with a substance which can be detected by a cell sorter, preferably a dye. Preferably, the dye is a fluorescent dye. A large number of different dyes are known in the art, including fluorescein, rhodamine, Texas red, phycoerythrin, and the like. Any detectable substance which has the appropriate characteristics for the cell sorter may be used (e.g., in the case of a fluorescent dye, a dye which can be excited by the sorter's light source, and an emission spectra which can be detected by the cell sorter's detectors).

In flow cytometry, a beam of laser light is projected through a liquid stream that contains cells, or other particles, which when struck by the focussed light give out signals which are picked up by detectors. These signals are then converted for computer storage and data analysis, and can provide information about various cellular properties. Cells labelled with a suitable dye are excited by the laser beam, and emit light at characteristic wavelengths. This emitted light is picked up by detectors, and these analogue signals are converted to digital signals, allowing for their storage, analysis and display.

Many larger flow cytometers are also "cell sorters", such as fluorescence-activated cell sorters (FACS), and are instruments which have the ability to selectively deposit cells from particular populations into tubes, or other collection vessels. In a particularly preferred embodiment, the cells are isolated using FACS. This procedure is well known in the art and described by, for example, Melamed, et al. Flow Cytometry and Sorting Wiley-Liss, Inc., New York, N.Y. (1990); Shapiro Practical Flow Cytometry, 4 ed, Wiley-Liss, Hoboken, NJ. (2003); and Robinson et al. Handbook of Flow Cytometry Methods Wiley-Liss, New York, N.Y (1993).

In order to sort cells, the instruments electronics interprets the signals collected for each cell as it is interrogated by the laser beam and compares the signal with sorting criteria set on the computer. If the cell meets the required criteria, an electrical charge is applied to the liquid stream which is being accurately broken into droplets containing the cells. This charge is applied to the stream at the precise moment the cell of interest is about to break off from the stream, then removed when the charged droplet has broken from the stream. As the droplets fall, they pass between two metal plates, which are strongly positively or negatively charged. Charged droplets get drawn towards the metal plate of the opposite polarity, and deposited in the collection vessel, or onto a microscope slide, for further examination.

The cells can automatically be deposited in collection vessels as single cells or as a plurality of cells, e.g. using a laser, e.g. an argon laser (488 nm) and for example with a Flow Cytometer fitted with an Autoclone unit (Coulter EPICS Altra, Beckman-Coulter, Miami, Fla., USA). Other examples of suitable FACS machines useful for the methods of the invention include, but are not limited to, MoFlo^{™} High-speed cell sorter (Dako-Cytomation Ltd), FACS Aria^{™} (Becton Dickinson), ALTRA^{™} Hyper sort (Beckman Coulter) and CyFlow^{™} sorting system (Partec GmbH).

For the selection of cells from a sample using solid-phase particles, any particle with the desired properties may be utilized. For example, large particles (e.g., greater than about 90-100 µm in diameter) may be used to facilitate sedimentation. Preferably, the particles are "magnetic particles" (i.e., particles which can be collected using a magnetic field). Typically, maternal cells labelled with the magnetic probe are passed through a column, held within a magnetic field. Labelled cells are retained on the column (held by the magnetic field), whilst unlabelled cells pass straight through and are eluted at the other end. Magnetic particles are now commonly available from a variety of manufacturers including Dynal Biotech (Oslo, Norway) and Miltenyi Biotech GmbH (Germany). An example of magnetic activated cell sorting (MACS) is provided by Al-Mufti *et al.* (1999) and US 4,675,286.

Laser-capture microdissection can also be used to select labelled cells. Methods of using laser-capture microdissection are known in the art (see, for example, U.S. 20030227611 and Bauer *et al.,* 2002).

As the skilled person will appreciate, maternal cells can be labelled with one type of label, and fetal cells with another type of label, and the respective cells types selected on the basis of the different labelling. For example, maternal cells can be labelled as described herein such that they produce a fluorescent green signal, and maternal cells can be labelled as described herein such that they produce a fluorescent red signal.

### Uses

Fetal cells comprise the same genetic DNA make up of the somatic cells of the fetus, and hence fetal cells isolated using the methods of the invention can be analysed for traits of interest and/or abnormalities of the fetus using techniques known in the art. Such analysis can be performed on any cellular material that enables the trait, or predisposition thereto, to be detected. Preferably, this material is nuclear DNA, however, at least in some instances it may be informative to analyse mitochondrial DNA, RNA or protein from the isolated fetal cells. Furthermore, the DNA may encode a gene, or may encode a functional RNA which is not translated, or the DNA analysed may even be an informative non-transcribed sequence or marker.

In one preferred embodiment, chromosomal abnormalities are detected. By "chromosomal abnormality" we include any gross abnormality in a chromosome or the number of chromosomes. For example, this includes detecting trisomy in chromosome 21 which is indicative of Down's syndrome, trisomy 18, trisomy 13, sex chromosomal abnormalities such as Klinefelter syndrome (47, XXY), XYY or Turner's syndrome, chromosome translocations and deletions. A small proportion of Down's syndrome patients have translocation and chromosomal deletion syndromes including Pradar-Willi syndrome and Angelman syndrome, both of which involve deletions of part of chromosome 15, and the detection of mutations (such as deletions, insertions, transitions, transversions and other mutations) in individual genes. Other types of chromosomal problems also exist such as Fragile X syndrome, hemophilia, spinal muscular dystrophy, myotonic dystrophy, Menkes disease and neurofibromatosis, which can be detected by DNA analysis.

The phrase "genetic abnormality" also refers to a single nucleotide substitution, deletion, insertion, micro-deletion, micro-insertion, short deletion, short insertion, multinucleotide substitution, and abnormal DNA methylation and loss of imprint (LOI). Such a genetic abnormality can be related to an inherited genetic disease such as a single-gene disorder (e.g., cystic fibrosis, Canavan, Tay-Sachs disease, Gaucher disease, Familial Dysautonomia, Niemann-Pick disease, Fanconi anemia, Ataxia telengectasia, Bloom syndrome, Familial Mediterranean fever (FMF), X-linked spondyloepiphyseal dysplasia tarda, factor XI), an imprinting disorder [e.g., Angelman Syndrome, Prader-Willi Syndrome, Beckwith-Wiedemann syndrome, Myoclonus-dystonia syndrome (MDS)], or to predisposition to various diseases (e.g., mutations in the BRCA1 and BRCA2 genes). Other genetic disorders which can be detected by DNA analysis are known such as thalassaemia, Duchenne muscular dystrophy, connexin 26, congenital adrenal hypoplasia, X-linked hydrocephalus, ornithine transcarbamylase deficiency, Huntington's disease, mitochondrial disorder, mucopolysaccharidosis I or IV, Norrie's disease, Rett syndrome, Smith-Lemli Optiz syndrome, 21-hydroxylase deficiency or holocarboxylase synthetase deficiency, diastrophic dysplasia, galactosialidosis, gangliosidosis, hereditary sensory neuropathy, hypogammaglobulinaemia, hypophosphatasia, Leigh's syndrome, aspartylglucosaminuria, metachromatic leukodystrophy Wilson's disease, steroid sulfatase deficiency, X-linked adrenoleukodystrophy, phosphorylase kinase deficiency (Type VI glycogen storage disease) and debranching enzyme deficiency (Type III glycogen storage disease). These and other genetic diseases are mentioned in The Metabolic and Molecular Basis of Inherited Disease, 8th Edition, Volumes I, II, III and IV, Scriver, C. R. et al. (eds), McGraw Hill, 2001. Clearly, any genetic disease where the gene has been cloned and mutations detected can be analysed.

The methods of the present invention can also be used to determine the sex of the fetus. For example, staining of the isolated fetal cells with a Y-chromosome specific marker will indicate that the fetus is male, whereas the lack of staining will indicate that the fetus is female.

In yet another use of the invention, the methods described herein can be used for paternity testing. Where the paternity of a child is disputed, the procedures of the invention enable this issue to be resolved early on during pregnancy. Many procedures have been described for parentage testing which rely on the analysis of suitable polymorphic markers. As used herein, the phrase "polymorphic markers" refers to any nucleic acid change (e.g., substitution, deletion, insertion, inversion), variable number of tandem repeats (VNTR), short tandem repeats (STR), minisatellite variant repeats (MVR) and the like. Typically, parentage testing involves DNA fingerprinting targeting informative repeat regions, or the analysis of highly polymorphic regions of the genome such as HLA loci.

### Analysis of Fetal Cells

Fetal cells processed and/or enriched using the methods of the invention can be analysed by a variety of procedures, however, typically genetic assays will be performed. Genetic assay methods include the standard techniques of karyotyping, analysis of methylation patterns, restriction fragment length polymorphism assays, sequencing and PCR-based assays (including multiplex F-PCR STR analysis, whole genome amplification and microarray analysis), as well as other methods described below.

Chromosomal abnormalities, either in structure or number, can be detected by karyotyping which is well known in the art such as FISH. Karyotyping analysis is generally performed on cells which have been arrested during mitosis by the addition of a mitotic spindle inhibitor such as colchicine. Preferably, a Giemsa-stained chromosome spread is prepared, allowing analysis of chromosome number as well as detection of chromosomal translocations.

The genetic assays may involve any suitable method for identifying mutations or polymorphisms, such as: sequencing of the DNA at one or more of the relevant positions; differential hybridisation of an oligonucleotide probe designed to hybridise at the relevant positions of either the wild-type or mutant sequence; denaturing gel electrophoresis following digestion with an appropriate restriction enzyme, preferably following amplification of the relevant DNA regions; S1 nuclease sequence analysis; non-denaturing gel electrophoresis, preferably following amplification of the relevant DNA regions; conventional RFLP (restriction fragment length polymorphism) assays; selective DNA amplification using oligonucleotides which are matched for the wild-type sequence and unmatched for the mutant sequence or vice versa; or the selective introduction of a restriction site using a PCR (or similar) primer matched for the wild-type or mutant genotype, followed by a restriction digest. The assay may be indirect, that is capable of detecting a mutation at another position or gene which is known to be linked to one or more of the mutant positions. The probes and primers may be fragments of DNA isolated from nature or may be synthetic.

A non-denaturing gel may be used to detect differing lengths of fragments resulting from digestion with an appropriate restriction enzyme. The DNA is usually amplified before digestion, for example using the polymerase chain reaction (PCR) method and modifications thereof.

Amplification of DNA may be achieved by the established PCR methods or by developments thereof or alternatives such as quantitative PCR, quantitative fluorescent PCR (QF-PCR), multiplex ligation dependent probe amplification, digital PCR, real time PCR (RT-PCR), single cell PCR, restriction fragment length polymorphism PCR (PCR-RFLP), PCR-RFLP/RT-PCR-RFLP, hot start PCR, nested PCR, *in situ* polonony PCR, *in situ* rolling circle amplification (RCA), bridge PCR, picotiter PCR and emulsion PCR. Other suitable amplification methods include the ligase chain reaction (LCR), transcription amplification, self-sustained sequence replication, selective amplification of target polynucleotide sequences, consensus sequence primed polymerase chain reaction (CP-PCR), arbitrarily primed polymerase chain reaction (AP-PCR), degenerate oligonucleotide-primed PCR (DOP-PCR) and nucleic acid based sequence amplification (NABSA). Other amplification methods that can be used herein include those described in US 5,242,794; 5,494,810; 4,988,617; and 6,582,938.

An "appropriate restriction enzyme" is one which will recognise and cut the wild-type sequence and not the mutated sequence or *vice versa*. The sequence which is recognised and cut by the restriction enzyme (or not, as the case may be) can be present as a consequence of the mutation or it can be introduced into the normal or mutant allele using mismatched oligonucleotides in the PCR reaction. It is convenient if the enzyme cuts DNA only infrequently, in other words if it recognises a sequence which occurs only rarely.

In another method, a pair of PCR primers are used which hybridise to either the wild-type genotype or the mutant genotype but not both. Whether amplified DNA is produced will then indicate the wild-type or mutant genotype (and hence phenotype).

A preferable method employs similar PCR primers but, as well as hybridising to only one of the wild-type or mutant sequences, they introduce a restriction site which is not otherwise there in either the wild-type or mutant sequences.

In order to facilitate subsequent cloning of amplified sequences, primers may have restriction enzyme sites appended to their 5' ends. Thus, all nucleotides of the primers are derived from the gene sequence of interest or sequences adjacent to that gene except the few nucleotides necessary to form a restriction enzyme site. Such enzymes and sites are well known in the art. The primers themselves can be synthesized using techniques which are well known in the art. Generally, the primers can be made using synthesizing machines which are commercially available.

PCR techniques that utilize fluorescent dyes may also be used to detect genetic defects in DNA from fetal cells isolated by the methods of the invention. These include, but are not limited to, the following five techniques.
i) Fluorescent dyes can be used to detect specific PCR amplified double stranded DNA product (e.g. ethidium bromide, or SYBR Green I).
ii) The 5' nuclease (TaqMan) assay can be used which utilizes a specially constructed primer whose fluorescence is quenched until it is released by the nuclease activity of the Taq DNA polymerase during extension of the PCR product.
iii) Assays based on Molecular Beacon technology can be used which rely on a specially constructed oligonucleotide that when self-hybridized quenches fluorescence (fluorescent dye and quencher molecule are adjacent). Upon hybridization to a specific amplified PCR product, fluorescence is increased due to separation of the quencher from the fluorescent molecule.
iv) Assays based on Amplifluor (Intergen) technology can be used which utilize specially prepared primers, where again fluorescence is quenched due to self-hybridization. In this case, fluorescence is released during PCR amplification by extension through the primer sequence, which results in the separation of fluorescent and quencher molecules.
v) Assays that rely on an increase in fluorescence resonance energy transfer can be used which utilize two specially designed adjacent primers, which have different fluorochromes on their ends. When these primers anneal to a specific PCR amplified product, the two fluorochromes are brought together. The excitation of one fluorochrome results in an increase in fluorescence of the other fluorochrome.

The acronym "FISH" references a technique that uses chromophore tags (fluorophores) that emit a secondary signal if illuminated with an excitation light to detect a chromosomal structure. FISH uses fluorescent probes which bind only to those parts of the chromosome with which they show a high degree of sequence similarity. Such tags may be directed to specific chromosomes and specific chromosome regions. The probe has to be long enough to hybridize specifically to its target (and not to similar sequences in the genome), but not too large to impede the hybridization process, and it should be tagged directly with fluorophores. This can be done in various ways, for example nick translation or PCR using tagged nucleotides. If signal amplification is necessary to exceed the detection threshold of the microscope (which depends on many factors such as probe labelling efficiency, the kind of probe and the fluorescent dye), secondary fluorescent tagged antibodies or streptavidin are bound to the tag molecules, thus amplifying the signal.

Fetal cells isolated using the methods of the invention can also be analysed using the MassARRAY® and SEQureDx^{™} procedures of Sequenom Technology (San Deigo, California, USA).

Fetal cells, or an enriched cell population of fetal cells, obtained using a method of the invention can be placed into wells of a microtitre plate (one cell per well) and analysed independently. Preferably, each cell will not only be screened for a trait(s) of interest, but screened to confirm/detect that the cell in a particular well is a fetal cell. In this instance, multiplex analysis can be performed as generally described by Findlay *et al.* (1996, 1998 and 2001).

The methods of the invention may include the step of fixing and permeabilizing the cells in the sample. Such procedures are known to those skilled in the art. For example, fixation may involve initial paraformaldehyde fixation followed by treatment with detergents such as Saponin, TWEEN-based detergents, Triton X-100, Nonidet NP40, NP40 substitutes, or other membrane disrupting detergents. Permeabilization may also involve treatment with alcohols (ethanol or methanol). Initial fixation may also be in ethanol. Combined fixation/permeabilization may also be performed using commercially available kits, including DAKO-Intrastain^{™}, Caltag's Fix & Perm reagents, Ortho Diagnostic's Permeafix^{™}. If required, methods for the extraction of DNA from fixed samples for genetic analysis are also known to those skilled in the art. For example, US 20040126796 discloses a method for the extraction of DNA from tissues and other samples, such as formalin-fixed tissue. The isolation of DNA from fixed samples for use in PCR has also been described by Lehman and Kreipe (2001) and Fitzgerald *et al.* (1993).

### Kits

Also disclosed herein is a kit for enriching fetal cells from a sample. In one example, the kit comprises at least two of the following;
i) an apparatus for obtaining a transcervical sample,
ii) an apparatus and/or media for transporting and/or storing the transcervical sample to a diagnostic laboratory,
iii) an apparatus for obtaining a second sample comprising maternal DNA but no fetal cell DNA from the mother,
iv) an apparatus and/or enzyme for treating the sample to produce at least a partial single cell suspension,
v) an apparatus for selecting cells based on cell size,
vi) an agent for positively selecting fetal cells,
vii) an agent for negatively selecting fetal cells, and/or
viii) a reagent(s) for performing a genetic assay.

In a preferred embodiment, the kit comprises
iv) an apparatus for at least partially mechanically disaggregating the sample, and
v) an apparatus for selecting cells based on cell size.

In another preferred embodiment, the kit comprises
iv) an apparatus for at least partially mechanically disaggregating the sample,
v) an apparatus for selecting cells based on cell size, and
vi) an agent for positively selecting fetal cells.

In another preferred embodiment, the kit comprises
iv) an apparatus for at least partially mechanically disaggregating the sample,
v) an apparatus for selecting cells based on cell size, and
vii) an agent for negatively selecting fetal cells.

In one embodiment, the agent(s) are each linked to magnetic beads.

Preferably, the apparatus for selecting cells based on cell size is a cell strainer.

Preferably, the agent for positively selecting fetal cells is an antibody.

Preferably, the agent for negatively selecting fetal cells is an antibody.

Also disclosed herein are kits for enriching fetal cells from a transcervical sample. In one example, the kit comprises at least two of the following;
i) an apparatus for obtaining the transcervical sample,
ii) an apparatus and/or media for transporting and/or storing the sample to a diagnostic laboratory,
iii) an apparatus for obtaining a second sample comprising maternal DNA but no fetal cell DNA from the mother,
iv) an apparatus and/or enzyme for treating the sample to produce at least a partial single cell suspension,
v) at least one apparatus for selecting multinucleated fetal cells using cell size, and/or
vi) a reagent(s) for performing a genetic assay.

In a preferred embodiment, the kit comprises
i) an apparatus for at least partially enzymatically disaggregating the sample, and
ii) at least one apparatus for selecting multinucleated fetal cells using cell size.

In a further embodiment, the kit comprises an apparatus for obtaining the sample, an apparatus and/or media for transporting and/or storing the sample to a diagnostic laboratory.

In an embodiment, the apparatus for selecting multinucleated fetal cells using cell size is two cell strainers with different mesh sizes.

Preferably, the apparatus for obtaining a second sample comprising maternal DNA but no fetal cell DNA from the mother is a mouth (buccal) swab.

In an embodiment, the enzyme is a collagenase, a protease or a combination thereof.

Preferably, the apparatus for transporting and/or storing the transcervical sample to the diagnostic laboratory is a tube which contains the transport and/or storage media to preserve the cells.

Preferably, the apparatus for obtaining a transcervical sample is a flexible aspiration catheter.

The kit may further comprise components for analysing the genotype of a fetal cell, determining the father of a fetus, and/or determining the sex of the fetus.

Typically, the kits will also include instructions recorded in a tangible form (e.g., contained on paper or an electronic medium), for example, for using a packaged agent for enriching fetal cells from a sample. The instructions will typically indicate the reagents and/or concentrations of reagents and at least one enrichment method parameter which might be, for example, the relative amounts of agents to use per amount of sample. In addition, such specifics as maintenance, time periods, temperature and buffer conditions may also be included.

### EXAMPLES

### Example 1 - Cell Preparation

A cervical mucus sample (transcervical sample) is collected using a fine, flexible aspiration catheter ("Aspiracath", Cook Australia; "Aspiration Kit", Medgyn) or a brush ("Tao brush endometrial sampler", Cook Australia). The aspiration catheter is inserted approximately 2-3cm into the cervix at the level of the internal os. A 0.5ml sample is collected by gentle aspiration (or if using an endometrial brush, by gentle rotation).

The catheter (or brush) is removed and the end of the device containing the sample is cut and placed in a sterile container for transport.

The sampling device is removed from the transport container using sterile forceps and transferred to an organ petri dish. The sample is washed from the device using 500 µl Phosphate Buffered Saline (PBS). Complete removal sometimes requires manual assistance using sterile forceps.

The sample is manually tweezed apart using sterile forceps into small pieces. The sample is further disaggregated by gentle pipetting using a 1ml pipette.

The entire sample is then passed through a cell strainer (100 µm Nylon Strainer sold by Becton Dickinson, USA) into a sterile 50ml FALCON^{™} tube. A further 3 ml PBS is passed through the strainer (making sure that all single cells have filtered through). The 50ml FALCON^{™} tube containing cells <100µm in size is centrifuged at 4000 rpm for 5 minutes and the cell pellet is resuspended in 1 ml PBS. An aliquot of the sample (thin layer) is placed onto a slide, air-dried and fixed. The slide is then stained with haematoxylin & eosin (H&E). Light microscopy is used to determine the presence/absence of syncytiotrophoblasts in the sample.

The portion of sample (Sample 1) passing through the strainer is largely a single cell suspension and is now appropriate for use in cell sorting procedures including MACS (for instance, see Example 3) and cell size selection (for instance, see Example 9). Sample 1 contains a mixture of cell types including Syncytiotrophoblasts, Cytotrophoblasts, and maternal cells.

The sample (Sample 2) retained on the strainer requires further treatment to render it as a single cell suspension. This can be done by:
- Placing the cell strainer upside down in a new sterile organ culture dish.
- Washing the sample from the strainer using 2 ml of liberase blendzyme 4 (Roche Diagnostics, USA, 0.5 WÜnsch units/ml) making sure that the entire sample trapped on the strainer is removed.
- Incubating the sample for 15-20 minutes at 37°C to disaggregate the sample into a single cell suspension.
- Centrifuging the sample at 1500 g for 2 min.
- Removing the supernatant (enzyme) and suspending the cells in 1ml PBS.
- Centrifuging the cells again at 1500 g for 2 minutes and resuspending the cells in 1 ml PBS.
- Placing a 100 µm cell strainer onto a 50 ml FALCON^{™} tube. Filtering the sample through a cell strainer (100µm) to remove any clumps of cells. Passing a further 2-3ml of PBS through the cell strainer (making sure that all single cells have filtered through).
- Centrifuging cells at 1500 g for 5 minutes and resuspending the cells in 500 µl PBS.
- Transferring the sample to a 1.5ml tube.
- Removing any cells remaining in the 50ml FALCON^{™} tube by washing the tube twice with 500 µl PBS and adding cells to the 1.5ml tube.

The portion of the sample (Sample 2) which did not pass through the strainer and which received an enzymic disaggregation treatment is now largely a single cell suspension and is now appropriate for use in a cell sorting procedure. This contains a mixture of cell types including Syncytiotrophoblasts, Cytotrophoblasts, and maternal cells. However, most of the Syncytiotrophoblasts appear in the sample, which passed through the cell strainer.

The two cell samples may be combined or treated separately.

### Example 2- Storage of Samples

Cells from Example 1 are centrifuged at 1500g for 5 min. The cell pellet is resuspended in 1 ml pre-cooled HTS-FRS medium and stored overnight or up to 3 days at 4°C with rotation.

### Example 3 - Enrichment of Fetal Cells Usine Magnetic Activated Cell Sorting (MACS)

### Positive selection of syncytiotrophoblasts using anti-NDOG1 antibody (2 step process)

Cells from Example 2 are centrifuged at 1500 g for 5 min. The cell pellet is washed with 1ml cold PBS containing 0.5% BSA and 0.5M EDTA, centrifuged and resuspended in 270 µl of cold PBS containing 0.5% BSA and 0.5M EDTA. 30 µl of rat serum (Sigma USA) is added to block non-specific binding. Cells are incubated for 10 minutes then centrifuged at 1500 g for 5 min. The cell pellet is resuspended in 270 µl of cold PBS containing 0.5% BSA and 0.5M EDTA.

30 µl of NDOG1 antibody (Serotec UK) is added and the cells incubated for 20 minutes at room temperature with rotation. The cells are washed twice with PBS containing 0.5% BSA and 0.5M EDTA to remove unbound antibody. The cells are then resuspended in 80 µl cold PBS containing 0.5% BSA and 0.5M EDTA.

20 µl of rat anti-mouse microbeads IgM (Miltenyi, Germany) are added. The cells are incubated for 20 minutes at room temperature with mixing every 5 minutes. The cells are then washed twice with PBS containing 0.5% BSA and 0.5M EDTA to remove unbound antibody.

Cell sorting is achieved using a pre-cooled large cell column (Miltenyi, Germany) using the following procedure;
- Place a MACS separation pre-cooled large cell column or LS cell column (Miltenyi Biotec) onto the separation unit (magnet).
- Place a sterile 15ml FALCON^{™} tube directly underneath the column.
- Prepare the column by rinsing it with 3ml of cold PBS containing 0.5% BSA and 0.5M EDTA. Once the entire amount of PBS has gone through the column, add a further 2ml of PBS containing 0.5% BSA and 0.5M EDTA to the column and wait until the column begins to elute PBS.
- Add 1ml of the cell suspension to the column. Collect the unlabelled cells, which pass through.
- Wash out the 1.5ml tube twice with 1ml PBS containing 2mM EDTA and 1% BSA to remove any cells remaining in tube and add them to the column.
- Once the column stops eluting buffer add a further 1ml of PBS containing 0.5% BSA and 0.5M EDTA to the column.
- Collect the total effluent. Discard the tube containing the unlabelled cell fraction.
- Remove the column from the separation unit and place it onto a new 15 ml FALCON^{™} collection tube.
- Pipette 3ml of cold PBS containing 0.5% BSA and 0.5M EDTA onto the column.
- Immediately flush out the fraction with the magnetically labelled cells by firmly applying the plunger supplied with the column.
- Remove the plunger and then add a further 2ml of PBS containing 0.5% BSA and 0.5M EDTA onto the column.
- Immediately flush out the fraction containing the magnetically labelled cells by firmly applying the plunger supplied with the column.
- Centrifuge the 15 ml tube containing "NDOG positive" cells for 5 min at 1500 g.

This is a trophoblast-enriched fraction, which is now available for further analysis using, for example, PCR (see Example 6) or FISH (see Example 7).

### Magnetic depletion of maternal cells using HLA negative selection (2 step process)

Cells from Example 2 are centrifuged at 1500 g for 5 min. The cell pellet is washed with 1ml cold PBS containing 0.5% BSA and 0.5M EDTA, centrifuged and resuspended in 300µl of cold PBS containing 0.5% BSA and 0.5M EDTA. Add 20 µl of biotinylated anti-HLA ABC antibody (Calbiochem USA). Incubate the cells on a rotor for 20 minutes at room temperature with rotation. Add 1ml of cold PBS containing 0.5% BSA and 0.5M EDTA to the cell suspension and centrifuge for 5 minutes at 1500 g. Remove the supernatant and wash the sample again in 1ml of PBS containing 0.5% BSA and 0.5M EDTA. Resuspend the cell pellet in 180µl of cold PBS containing 0.5% BSA and 0.5M EDTA.

Add 20µl of Streptavidin Microbeads (Miltenyi). Incubate the cells with rotation for 15 minutes at 4°C with rotation. Add 1ml of PBS containing 0.5% BSA and 0.5M EDTA to the cell suspension and centrifuge for 5 min at 1500 g. Remove the supernatant and wash the sample again in 1 ml of PBS containing 0.5% BSA and 0.5M EDTA. Remove the supernatant. Resuspend the pellet in 1ml of PBS containing 0.5% BSA and 0.5M EDTA.

Cell sorting is achieved using a pre-cooled large cell column (Miltenyi, Germany) using the following procedure;
- Place a MACS separation pre-cooled large cell column or LS cell column (Miltenyi Biotec) onto the separation unit (magnet).
- Place a sterile 15ml FALCON^{™} tube directly underneath the column.
- Prepare the column by rinsing it with 3ml of cold PBS containing 0.5% BSA and 0.5M EDTA. Once the entire amount of PBS has gone through the column, add a further 2ml of PBS containing 0.5% BSA and 0.5M EDTA to the column and wait until the column begins to elute PBS.
- Add 1 ml of cell suspension to the column. Collect the unlabelled cells which pass through.
- Wash out the 1.5ml tube with 1ml of PBS containing 2mM EDTA and 1% BSA to remove any cells remaining in tube and add them to the column.
- Once the column stops eluting buffer add a further 1 ml of PBS containing 0.5% BSA and 0.5M EDTA to the column.
- Collect the total effluent.

This is a trophoblast-enriched fraction which is now available for further analysis using, for example, PCR (see Example 6) or FISH (see Example 7).

### Example 4 - Comparison of Fetal Cell Retrieval Using Different Samplers

- 160 samples were used in this study.
- Samples were collected from women undergoing elective termination of pregnancy using different sampling devices.
- Three types of samplers were investigated:
   i. Medgyn catheters
   ii. Aspiracaths
   iii. Tao brush endometrial sampler
- Current maximum syncytial cell retrieval rate obtained using an aspiration device is 60-80%.
- The highest percentage of syncytial cells is consistently observed in aspiration samples containing cervical mucus (74-83%) (Table 1).

**Table 1: Current syncytial cell recovery rate using different sampling devices.**

| **Sampling Device** | **Overall syncytial cell retrieval rate (%)** | **Total number of samples** | **% of syncytial cell positive samples containing cervical mucus** |
|---|---|---|---|
| Medgyn Catheters | 80 | 15 | 83 |
| Aspiracaths | 60 | 57 | 74 |
| Tao brush endometrial sampler | 17 | 88 | 31 |

### Example 5 - Influence Of Gestational Age On Syncytial Cell Retrieval

- 72 samples were used in this study.
- Samples were collected from pregnant women between 5-12 weeks gestation using either a Aspiration kit (Medgyn) or an Aspiracath (Cook Australia)
- The majority of samples collected were between 6-8 weeks gestation.
- Syncytial cells were observed as early as 5-6 weeks gestation.
- No significant differences were evident between the different gestational age groups due to the low numbers in each group to perform suitable statistical analysis (Table 2).

**Table 2: Influence of gestational age on syncytial cell recovery.**

| **Sampling Device** | **Syncytial cell retrieval at different gestational ages (%)** | | | | | | |
|---|---|---|---|---|---|---|---|
| | **5** | **6** | **7** | **8** | **9** | **10** | **12** |
| Aspiration Kit | N/A | 0 | 83 | 50 | 100 | 100 | 100 |
| Aspiracath | 100 | 41 | 70 | 70 | 0 | 100 | N/A |
| **Average %** | **100** | **39** | **72** | **67** | **50** | **100** | **100** |
| **Number of samples** | 1 | 18 | 32 | 12 | 4 | 4 | 1 |

### Example 6 - Fluorescent Multiplex PCR Analysis

36 NDOG positive or HLA negative samples were used in this study. All samples were subjected multiplex QF-PCR using four short tandem repeat (STR) markers on chromosome 21 (D21S11, D21S1413, D21S1437 and D21S1442) and two sex chromosome markers (hypoxanthine guanine phosphoribosyl transferase (HPRT) and amelogenin X and Y) to simultaneously determine the sex of the isolated syncytiotrophoblasts.

STR profiles were derived following analysis of the PCR products on a 3130 Genetic Analyser (Applied Biosystems) using Genescan version 3.7 software. Comparison of the fetal and maternal STR profiles confirmed fetal origin and the presence or absence of maternal cell contamination. Cells of fetal origin were expected to show two fluorescent peaks for each STR marker allele, one shared with the mother and the other inherited from the father.

Allelic peak ratios for each marker were also calculated based on the amount of DNA produced in the PCR amplification. Normal heterozygotes were expected to show ratios of the fluorescent intensity of the two peaks close to 1:1. In any cases of Down syndrome, chromosome 21 STR markers were expected to either display a triallelic pattern (1:1:1 ratio) or a di-allelic pattern (2:1).

### Criteria for interpretation of genotyping profiles:

1. Allele dosage ratio between 0.8 to 1.4 is defined as normal (disomic)
2. Allele dosage ratio greater than 1.8, less than 0.65 or the presence of three alleles is defined as abnormal (trisomic).
3. Anything between 1.4 and 1.8 is considered non-informative.
4. A minimum of three informative markers is required for confident interpretation, owing to the possibility of primer site polymorphisms and somatic repeat instability.
5. A single peak is considered non-informative.

The results can be summarized as follows:
- Of these 36 samples, 20 (44%) were shown to contain syncytiotrophoblasts by morphology in H&E stained smears.
- Samples containing no fetal cells exhibited a maternal DNA profile as expected.
- 55% of the fetal cell positive samples exhibited a pure fetal DNA profile ("clean fingerprint") (Table 3).
- The current rate range for the isolation of pure fetal DNA profiles is 85% - 99.4% (mean of 94.5%).
- 30% of samples exhibit a mixed profile consisting of both a maternal and fetal DNA.
- Both sorting procedures allow pure fetal DNA profiles to be obtained as assessed by PCR (Table 4). NDOG1 positive selection performs slightly better than HLA negative selection. However, there were two samples (numbers 39 & 42) which gave a pure fetal DNA profile with HLA but not with NDOG1.
- Fetal cell purity >90% (according to the FISH data) is essential to provide "clean" fingerprints for genotyping (Figure 1).

**Table 3: DNA profiles of samples shown to contain syncytiotrophoblasts following PCR STR analysis.**

| | **Pure Fetal** | **DNA Profiling Results Mixed (Fetal + Maternal)** | **Maternal** |
|---|---|---|---|
| **% of samples exhibiting a different profile** | 55% | 30% | 15% |
| **Number of samples** | 11 | 6 | 3 |

**Table 4: Comparison of DNA profiles: NDOG1 (PBS) versus HLA (LIB) sorted samples.**

| **TCC Sample Number** | **Sorting Procedure NDOG1 positive selection** | **HLA negative selection** |
|---|---|---|
| DH13 | Pure fetal # | Maternal |
| DH15 | Pure fetal # | Maternal |
| DH16 | Pure fetal | Pure fetal |
| DH17 | Pure fetal # | Mixed |
| DH18 | Mixed | Mixed |
| DH28 | Pure fetal | Pure fetal |
| DH33 | Pure fetal | Pure fetal |
| DH37 | Mixed | Mixed |
| DH38 | Mixed | Mixed |
| DH39 | Mixed | Pure fetal * |
| DH42 | Mixed | Pure fetal * |
| DH43 | Maternal | Maternal |
| DH44 | Maternal | Maternal |
| DH45 | Mixed | Mixed |
| DH47 | Maternal | Maternal |
| DH48 | Maternal | Maternal |
| DH50 | Maternal | Maternal |
| DH56 | Maternal | Maternal |
| DH62 | Maternal | Maternal |
| DH65 | Maternal | Maternal |
| DH68 | Maternal | Maternal |
| DH69 | Maternal | Maternal |
| DH77 | Mixed | Mixed |
| DH78 | Maternal | Maternal |
| DH81 | Maternal | Maternal |

| | | |
|---|---|---|
| * HLA sorted, liberase treated fraction gave a better DNA profile by PCR. # NDOG sorted, PBS treated fraction gave a better DNA profile by PCR | | |

### Example 7 - Fluorescent In-Situ Hybridisation Analysis

### NDOG positive sorted

85 PBS treated samples (refer to Example 1, Sample 1) were subjected to FISH analysis.
- 46 out of the 85 samples (54%) tested had syncytiotrophoblasts cells present.
- Of the 46 positive fetal cells samples investigated, 29 (63%) were shown to be male by FISH.
- The average number of male fetal nuclei observed was more than 20,000.
- The number of male fetal nuclei varied from 5 nuclei up to 240,675 nuclei per sample.
- No significant differences were detected in the number of nuclei between each gestational time point because the majority of male samples were at 7 weeks gestation. (Table 5).
- 55% of the male samples had nuclei purity levels of greater than 90% (Table 6).
- 21% of the male samples had nuclei purity levels between 60-89%.
- 24% of the male samples had nuclei purity levels less than 59%.
- Figure 2 shows an example of a multinucleated syncytiotrophoblast from a NDOG1 positive sorted sample.

**Table 5: Average number of male fetal nuclei at different gestational ages using FISH analysis.**

| | **Gestational Age** | | | | | |
|---|---|---|---|---|---|---|
| | **4-5** | **6** | **7** | **8** | **9** | **10** |
| **Number of male fetal nuclei/ sample** | 902 | 8,591 | 33,681 | 1,494 | 8,665 | 3,660 |
| **Range** | 265-1,540 | 5-28,668 | 10-240,675 | 219-3,132 | 520-15,230 | N/A |
| **Number of samples** | 2 | 5 | 15 | 3 | 3 | 1 |

**Table 6: Percentage purity of male NDOG1 positive sorted nuclei following FISH analysis.**

| | **% Purity** | | | | |
|---|---|---|---|---|---|
| | **>90** | **80-89** | **70-79** | **60-69** | **<59** |
| **Average** | 97 | 88.5 | 76.9 | 62.7 | 16.2 |
| **Range** | 93-99.84 | 87.89-89.04 | 75.43-78.34 | 61.4-63.93 | 4.17-43.86 |
| **Number of samples** | 16 | 2 | 2 | 2 | 7 |

### HLA negative sorted

42 samples treated with liberase (refer to Example 1, Sample 2) were subjected to FISH analysis
- 30 out the 42 samples (71%) tested had syncytiotrophoblasts present.
- Of the 30 fetal cell positive samples, 16 samples (53%) were shown to be male by FISH.
- The average number of male fetal nuclei observed more than 38,000.
- The number of male fetal nuclei varied from 30 nuclei up to 250,000 nuclei per sample.
- 56% of male nuclei samples had purity levels of greater than 90% (Table 7).
- Figure 3 shows an example of multiple male fetal nuclei observed from a HLA negative sorted sample.

**Table 7: Percentage purity of male HLA sorted nuclei following FISH analysis.**

| | **% Purity** | | | | |
|---|---|---|---|---|---|
| | **>90** | **80-89** | **70-79** | **60-69** | **<59** |
| **Average** | 98.67 | N/A | N/A | 68.67 | 13.02 |
| **Range** | 95.5 - 100 | N/A | N/A | N/A | 4.1-38.68 |
| **Number of samples** | 9 | 0 | 0 | 1 | 6 |

### Example 8 - Estimated Cell Loss Following Storage

- 18 samples were processed and stored with a preservative/cyropreservative agent (i.e. HTS-FRS media or CryoStor CS5)
- Cell smears were stained with diamidino-2-phenylindonle (DAPI) and the number of nuclei was counted.
- 23% cell loss was recorded with HTS-FRS media (n=10) (Table 8).
- 36% cell loss was recorded with CryoStor CS5 (n=8)

**Table 8: Estimated cell loss following storage with HTS-FRS or CryoStore CS5 media.**

| **Sample Number** | **Number of nuclei (pre-storage)** | **Number of nuclei (after 24 hrs/ after freezing)** | **% cell loss** |
|---|---|---|---|
| **HTS-FRS media** | | | |
| DH165 | 52958 | 37198 | 29.8 |
| DH166 | 15779 | 106197 | 32.7 |
| DH167 | 66483 | 50379 | 24.2 |
| DH168 | 75559 | 72227 | 4.4 |
| DH169 | 52701 | 38962 | 26.1 |
| DH171 | 51806 | 40627 | 21.6 |
| DH172 | 146240 | 97752 | 33.2 |
| DH175 | 16370 | 11769 | 28.1 |
| DH176 | 75561 | 72922 | 3.5 |
| DH187 | 45231 | 34689 | 23.31 |
| **Average % cell loss** | | **23+11 (SD)** | |

| **CrvoStore CS5** | | | |
|---|---|---|---|
| DH152 | 13722 | 9498 | 30.8 |
| DH153 | 15361 | 7954 | 48.2 |
| DH154 | 10280 | 9378 | 8.8 |
| DH155 | 45185 | 34315 | 24.1 |
| DH157 | 16915 | 15110 | 10.7 |
| DH159 | 20429 | 7077 | 65.4 |
| DH160 | 26732 | 12312 | 53.9 |
| DH164 | 11613 | 5999 | 48.3 |
| **Average % cell loss** | | **36+21** | |

### Example 9 -Enrichment of Fetal Cells (Syncytiotrophoblasts) Based on Cell Size (One Step Process)

The sample is prepared according to the method described in Example 1 (referred to as Sample 1) with some minor modifications. Briefly, the sample is manually dissagregated, then passed through a cell strainer of 100µm in mesh size into a 50ml FALCON^{™} tube.

Cells <100 µm in size are subsequently passed through a second cell strainer 40 or 70 µm in size into a 50 ml FALCON^{™} tube. A further 3-ml PBS is passed through the strainer (making sure that all single cells have filtered through). The portion of sample passing through the strainer comprises mainly of extra villous cytotrophoblasts, intra villous cytotrophoblasts and maternal cells (<40µm in size). The portion of sample (Sample 3) remaining on the cell strainer comprises mainly of syncytiotrophoblasts (<100, >40 or <100, >70 µm in size).

The cell strainer is carefully removed from the 50 ml FALCON^{™} tube and placed upside down in a new sterile organ culture dish. The sample is washed from the cell strainer using 2 ml of PBS making sure that the entire sample trapped on the strainer is removed. Cells <100, >40 or <100, >70 µm in size are transferred into a 1.5ml tube and centrifuged at 4000 rpm for 5 minutes. The cell pellet is resuspended in 20-100 µl PCR grade water (volume is dependent on the size of the pellet following size selection). This is a syncytiotrophoblast-enriched fraction, which is now available for further analysis using, for example, FISH (Figure 4) or QF-PCR (Figure 5).

Briefly,
■ 24 pre-selected samples shown to contain syncytiotrophoblasts by morphology were used in this study.
■ Size range varied from 40-100 µm for the isolation of pure populations of syncytiotrophoblasts (Table 9).

50% (12/24) of samples tested exhibited a pure fetal DNA profile ("clean fingerprint") regardless of the size selection (Table 10). 25% (7/24) of samples tested exhibited a mixed DNA profile (fetal + maternal) regardless of the size selection (Table 10). All samples exhibiting a pure profile were shown to be from a chromosome 21 disomic fetus.

### Example 10 - Enrichment of Syncytiotrophoblasts Using Two Step Process

A syncytiotrophoblast-enriched fraction is prepared as outlined in Example 9, using size selection. The Cells <100, >40 µm in size are then transferred into a 1.5ml tube and centrifuged at 4000 rpm for 5 minutes. The cell pellet is resuspended in 100 µl PBS.

Cells are centrifuged at 1500 g for 5 min. The cell pellet is washed with 1 ml cold PBS containing 0.5% BSA and 0.5M EDTA, centrifuged and resuspended in 270 µl of cold PBS containing 0.5% BSA and 0.5M EDTA.

30 µl of NDOG1 antibody (Serotec UK) is added and the cells incubated for 20 minutes at room temperature with rotation. The cells are washed twice with PBS containing 0.5% BSA and 0.5M EDTA to remove unbound antibody. The cells are then resuspended in 80 µl cold PBS containing 0.5% BSA and 0.5M EDTA.

**Table 9: DNA profiling results using different size cell strainers to isolate syncytiotrophoblasts.**

| **Sample Number *** | **Cell size (µm)** | | |
|---|---|---|---|
| | **<100, >70** | **<100, >40** | **<70, >40** |
| DH46 | N/A | Maternal | N/A |
| DH53 | N/A | Pure | N/A |
| DH205 | N/A | Pure | N/A |
| DH206 | N/A | Mixed | N/A |
| DH207 | N/A | Pure | N/A |
| DH208 | N/A | Maternal | N/A |
| DH210 | Mixed | N/A | No amplification |
| DH211 | Mixed | N/A | Pure |
| DH213 | Mixed | N/A | Mixed |
| DH215 | Pure | N/A | No amplification |
| DH217 | Maternal | N/A | Maternal |
| DH218 | Maternal | Mixed | N/A |
| DH228 | Pure | Mixed | Maternal |
| DH237 | N/A | Pure | N/A |
| DH239 | N/A | Mixed | N/A |
| DH240 | N/A | Mixed | N/A |
| DH242 | N/A | Maternal | N/A |
| DH266 | N/A | Mixed # | N/A |
| DH283 | N/A | Pure | N/A |
| DH285 | N/A | Maternal | N/A |
| DH292 | Pure | Pure | Pure |
| DH565 | N/A | Pure | N/A |
| DH567 | N/A | Pure | N/A |
| DH568 | N/A | Pure | N/A |

| | | | |
|---|---|---|---|
| * Samples have been pre-selected to only include syncytial fetal positive samples; ^{#} Isolated cells mainly fetal (allelic ratio's not affected by maternal contamination are within the normal range of 0.8 to 1.4) | | | |

**Table 10: Summary of results using size selection to isolate syncytiotrophoblasts.**

| **Number of samples (pre-selected to only include fetal positive samples)** | **Cell purity following size selection** | | |
|---|---|---|---|
| | **Pure fetal** | **Mixed (fetal+maternal)** | **Maternal** |
| 24 | 50% | 25% | 21% |

20 µl of rat anti-mouse microbeads IgM (Miltenyi, Germany) are added. The cells are incubated for 20 minutes at room temperature with mixing every 5 minutes. The cells are then washed twice with PBS containing 0.5% BSA and 0.5M EDTA to remove unbound antibody.

Cell sorting is achieved using a pre-cooled large cell column (Miltenyi, Germany) using the following procedure;
- Place a MACS separation pre-cooled large cell column or LS cell column (Miltenyi Biotec) onto the separation unit (magnet).
- Place a sterile 15ml FALCON^{™} tube directly underneath the column.
- Prepare the column by rinsing it with 3ml of cold PBS containing 0.5% BSA and 0.5M EDTA. Once the entire amount of PBS has gone through the column, add a further 2ml of PBS containing 0.5% BSA and 0.5M EDTA to the column and wait until the column begins to elute PBS.
- Add 1ml of the cell suspension to the column. Collect the unlabelled cells, which pass through.
- Wash out the 1.5ml tube twice with 1ml PBS containing 2mM EDTA and 1% BSA to remove any cells remaining in tube and add them to the column.
- Once the column stops eluting buffer add a further 1ml of PBS containing 0.5% BSA and 0.5M EDTA to the column.
- Collect the total effluent. Discard the tube containing the unlabelled cell fraction.
- Remove the column from the separation unit and place it onto a new 15 ml FALCON^{™} collection tube.
- Pipette 3ml of cold PBS containing 0.5% BSA and 0.5M EDTA onto the column.
- Immediately flush out the fraction with the magnetically labelled cells by firmly applying the plunger supplied with the column.
- Remove the plunger and then add a further 2ml of PBS containing 0.5% BSA and 0.5M EDTA onto the column.
- Immediately flush out the fraction containing the magnetically labelled cells by firmly applying the plunger supplied with the column.
- Centrifuge the 15 ml tube containing "NDOG positive" cells for 5 min at 1500 g.

This is a syncytiotrophoblast-enriched fraction, which is now available for further analysis using, for example, FISH or PCR.

The 2 step process allows the sample to be further purified in those instances where size selection alone does not yield a fetal enriched sample.

An example of this is shown in Figure 6. Size selection (one step process) (Figure 6a) and MACS using NDOG1 positive selection (Figure 6b) exhibited a mixed DNA profile (Figure 6c), however, when used in combination (two step process) a pure DNA profile ("clean fingerprint") was obtained for genotyping (Figure 6d). The isolated syncytiotrophoblasts were shown to be from a male disomic chromosome 21 fetus (Table 11).

**Table 11: Pattern of inheritance of isolated fetal cells from mother to fetus with calculated allelic ratios for each individual locus.**

| **Sample Type** | **Sex chromosome markers** | | | | **Chromosome 21 markers** | | | | | | | |
|---|---|---|---|---|---|---|---|---|---|---|---|---|
| | **Amelogenin** | | **HPRT** | | **D21S1437** | | **D21S1413** | | **D21S11** | | **D21S1442** | |
| | Size | Allele ratio | Size | Allele ratio | Size | Allele ratio | Size | Allele ratio | Size | Allele ratio | Size | Allele ratio |
| Maternal DNA | **104** | | 276 | 1.1 | **136** | | **175** | 1.2 | **237** | 1.2 | 243 | 1.0 |
| | | | **280** | | | | 179 | | 241 | | **251** | |
| Isolated fetal cells | **104** | 0.8 | **280** | | **136** | 1.3 | 154 | 0.8 | 219 | 1.1 | 235 | 1.0 |
| | **109** | | | | 149 | | **175** | | **237** | | **251** | |

| | | | | | | | | | | | | |
|---|---|---|---|---|---|---|---|---|---|---|---|---|
| Numbers indicate the length of amplified sequences in base pairs. Alleles shared between fetal and maternal DNA are shown in bold. The example of fetal cells is from a male disomic chromosome 21 fetus. The syncytiotrophoblasts were enriched using two step process (size selection + MACS using NDOG1 positive selection) (Figure 6d). any or all of these matters form part of the prior art base or were common general knowledge in the field relevant to the present invention as it existed before the priority date of each claim of this application. | | | | | | | | | | | | |

### REFERENCES

Adinolphi and Sherlock (1997) Hum. Reprod. Update. 3: 383-392.
Adinolphi et al. (1995a) Prenat. Diagn 15: 35-39.
Adinolphi et al. (1995b) Prenat. Diagn. 15: 943-949.
Al-Mufti et al. (1999) Am. J. Med. Genet. 85:66-75.
Bauer et al. (2002) Int. J. Legal Med. 116:39-42.
Bischoff and Simpson (2006) 18:206-220.
Blaschitz et al. (2000) Placenta 21:733-741.
Bulmer et al (1995) Prenat. Diagn 15: 1143-1153.
Bussani et al. (2002) Prenat. Diagn. 22: 1098-1101.
Bussani et al. (2004) Mol. Diagn. 8:259-63.
Bussani et al. (2007) Mol. Diagn. Ther. 11:117-121.
Cioni et al. (2003) Prenat. Diagn 23: 168-171.
Daryanii et al. (1997) Prenat. Diagn. 17: 243-248.
Fejgin et al. (2001) Prenat. Diagn. 21: 619-621.
Findlay et al. (1996) Hum Reprod. Update 2: 137-152.
Findlay et al. (1998) J Clin. Pathol. Mol Pathol. 51: 164-167.
Findlay et al. (2001) Mol Cellul. Endocrinol. 183: S5-S12.
Fitzgerald et al. (1993) Biotechniques 15:128-133.
Goldberg et al. (1980) Am J Obstet. Gynecol. 138:436-440.
Harrington et al. (1997) Science 275: 973-977.
Inglis et al. (2008) J Immunological Methods 329 151-156.
Katz-Jaffe et al. (2005) BJOG 112: 595-600.
Kingdom et al. (1995) Obstet. Gynecol. 86: 283-288.
Lehman and Kreipe (2001) Methods 25:409-418.
Mantzaris et al. (2005) Aus. NZ J Obstet. Gynaecol. 45: 529-532.
Massari et al. (1996) Hum. Genet. 97: 150-155.
Michalet et al. (2005) Science 307:538-544.
Miller et al. (1999) Hum. Reprod. 14: 521-531.
Murthy et al. (2006) Biomed. Microdevices 8:231-237.
Rhine et al. (1975) Am J Obste. Gynecol. 122: 155-160.
Rhine et al. (1977) Birth Defects Orig. Artic. Ser. 13: 231-247.
Rodeck et al. (1995). Prenat. Diagn. 15: 933-942.
Seimiya et al. (2000) EMBO J 19: 2652-2661.
Shettles (1971) Nature 230: 52-53.
Tokumasu and Dvorak (2003) J. Microsc. 211:256-261.
Tutschek et al. (1995) Prenat. Diagn. 15: 951-960.
Vaziri et al. (1994) PNAS 91:9857-9860.
Warren et al. (1972) Am. J Hum. Genet. 24: 22
Wu et al. (2007) J Micromech. Microeng. 17:1992-1999.

## Claims

1. A method of enriching multinucleated fetal cells from a transcervical sample from a pregnant female, the method comprising selecting cells which are between about 40µm and 150µm in size.

2. The method of claim 1, wherein the method comprises treating the sample to produce at least a partial single cell suspension before the cells are selected.

3. The method of claim 1 which comprises
i) at least partially mechanically disaggregating the sample to produce at least a partial single cell suspension,
ii) filtering the at least partial single cell suspension through a first cell strainer which has a mesh size of about 100µM and collecting the cells that pass through the first cell strainer, and
iii) filtering the cells collected in step ii) through a second cell strainer which has a mesh size of about 40µM and collecting the cells that did not pass through the second cell strainer.

4. The method of claim 1 which comprises
i) at least partially enzymatically disaggregating the sample to produce at least a partial single cell suspension, and
ii) filtering the at least partial single cell suspension through a cell strainer which has a mesh size of about 40µM and collecting the cells that did not pass through the cell strainer.

5. The method of claim 1 which comprises
i) at least partially mechanically disaggregating the sample to produce at least a partial single cell suspension,
ii) filtering the at least partial single cell suspension through a cell strainer which has a mesh size of about 100µM and collecting the cells that pass through the first cell strainer, and
iii) sorting the cells collected in step ii) by fluorescent activated cell separation (FACS) based on forward scatter and collecting cells which are at least about 40µM in size.

6. The method of claim 1 which comprises
i) treating the sample to produce at least a partial single cell suspension, and
ii) sorting the at least partial single cell suspension by fluorescent activated cell separation (FACS) based on forward scatter and collecting cells which are between about 40µm and 100µm in size.

7. A method for processing a transcervical sample from a pregnant female, the method comprising
i) treating the sample to produce at least a partial single cell suspension,
ii) selecting cells which are between about 40µm and 150µm in size,
wherein at least some of the cells obtained from step ii) are fetal cells which are suitable for genetic analysis and/or enrichment.

8. The method of claim 7, wherein step i) comprises treating the sample by at least partially mechanically disaggregating the sample, and/or treating the sample by at least partially enzymatically disaggregating the sample.

9. The method of claim 7 or claim 8, wherein step ii) comprises filtering the product of step i) through a cell strainer and collecting the cells that pass through the cell strainer.

10. The method of claim 9 which further comprises
iii) treating material which did not pass through the cell strainer with at least one enzyme capable of disassociating aggregated cells, and
iv) selecting cells obtained from step iii) based on cell size.

11. A method of enriching fetal cells from a transcervical sample from a pregnant female, the method comprising
i) processing a transcervical sample according to the method of any one of claims 7 to 10, and
ii) positively and/or negatively selecting fetal cells.

12. A method for analysing the genotype of a fetal cell at a locus of interest, the method comprising
i) processing a transcervical sample using a method according to any one of claims 7 to 10 and/or enriching fetal cells using a method according to any one of claims 1 to 6 or 11, and
ii) analysing the genotype of at least one fetal cell at a locus of interest.

13. A method of determining the sex of a fetus, the method comprising
i) processing a transcervical sample using a method according to any one of claims 7 to 10 and/or enriching fetal cells using a method according to any one of claims 1 to 6 or 11, and
ii) analysing at least one fetal cell to determine the sex of the fetus.

14. A method of determining the father of a fetus, the method comprising
i) processing a transcervical sample using a method according to any one of claims 7 to 10 and/or enriching fetal cells using a method according to any one of claims 1 to 6 or 11, and
ii) determining the genotype of the candidate father at one or more loci,
iii) determining the genotype of the fetus at one or more of said loci, and
iv) comparing the genotypes of ii) and iii) to determine the probability that the candidate father is the biological father of the fetus.

## Patentansprüche

1. Verfahren zum Anreichern vielkerniger fetaler Zellen aus einer transzervikalen Probe einer schwangeren Frau, wobei das Verfahren das Selektieren von Zellen, die zwischen etwa 40 µm und 150 µm groß sind, umfasst.

2. Verfahren gemäß Anspruch 1, wobei das Verfahren das Behandeln der Probe umfasst, um wenigstens eine teilweise einzelzellige Suspension herzustellen, bevor die Zellen selektiert werden.

3. Verfahren gemäß Anspruch 1, umfassend
i) wenigstens teilweises mechanisches Disaggregieren der Probe, um wenigstens eine teilweise einzelzellige Suspension herzustellen,
ii) Filtrieren der wenigstens teilweise einzelzelligen Suspension durch ein erstes Zellsieb, das eine Maschengröße von etwa 100 µM aufweist, und Sammeln der Zellen, die durch das erste Zellsieb passieren, und
iii) Filtrieren der in Schritt ii) gesammelten Zellen durch ein zweites Zellsieb, das eine Maschengröße von etwa 40 µM aufweist, und Sammeln der Zellen, die nicht durch das zweite Zellsieb passiert sind.

4. Verfahren gemäß Anspruch 1, umfassend
i) wenigstens teilweises enzymatisches Disaggregieren der Probe, um wenigstens eine teilweise einzelzellige Suspension herzustellen, und
ii) Filtrieren der wenigstens teilweise einzelzelligen Suspension durch ein Zellsieb, das eine Maschengröße von etwa 40 µM aufweist, und Sammeln der Zellen, die nicht durch das Zellsieb passiert sind.

5. Verfahren gemäß Anspruch 1, umfassend
i) wenigstens teilweises mechanisches Disaggregieren der Probe, um wenigstens eine teilweise einzelzellige Suspension herzustellen,
ii) Filtrieren der wenigstens teilweise einzelzelligen Suspension durch ein Zellsieb, das eine Maschengröße von etwa 100 µM aufweist, und Sammeln der Zellen, die durch das erste Zellsieb passieren, und
iii) Sortieren der in Schritt ii) gesammelten Zellen durch fluoreszenzaktivierte Zelltrennung (FACS) auf der Grundlage von Vorwärtsstreuung und Sammeln von Zellen, die wenigstens etwa 40 µM groß sind.

6. Verfahren gemäß Anspruch 1, umfassend
i) Behandeln der Probe, um wenigstens eine teilweise einzelzellige Suspension herzustellen, und
ii) Sortieren der wenigstens teilweise einzelzelligen Suspension durch fluoreszenzaktivierte Zelltrennung (FACS) auf der Grundlage von Vorwärtsstreuung und Sammeln von Zellen, die zwischen etwa 40 µM und 100 µM groß sind.

7. Verfahren zum Verarbeiten einer transzervikalen Probe einer schwangeren Frau, wobei das Verfahren umfasst:
i) Behandeln der Probe, um wenigstens eine teilweise einzelzellige Suspension herzustellen,
ii) Selektieren von Zellen, die zwischen etwa 40 µM und 150 µM groß sind, wobei wenigstens manche der in Schritt ii) erhaltenen Zellen fetale Zellen sind, die für genetische Analyse und/oder Anreicherung geeignet sind.

8. Verfahren gemäß Anspruch 7, wobei Schritt i) Behandeln der Probe durch wenigstens teilweises mechanisches Disaggregieren der Probe und/oder Behandeln der Probe durch wenigstens teilweises enzymatisches Disaggregieren der Probe umfasst.

9. Verfahren gemäß Anspruch 7 oder Anspruch 8, wobei Schritt ii) Filtrieren des Produkts von Schritt i) durch ein Zellsieb und Sammeln der Zellen, die durch das Zellsieb passieren, umfasst.

10. Verfahren gemäß Anspruch 9, ferner umfassend
iii) Behandeln von Material, das nicht durch das Zellsieb passiert hat, mit wenigstens einem Enzym, das zum Disassoziieren aggregierter Zellen fähig ist, und
iv) Selektieren von in Schritt iii) erhaltenen Zellen auf der Grundlage der Zellgröße.

11. Verfahren zum Anreichern fetaler Zellen aus einer transzervikalen Probe einer schwangeren Frau, wobei das Verfahren
i) Verarbeiten einer transzervikalen Probe gemäß dem Verfahren eines der Ansprüche 7 bis 10 und
ii) positives und/oder negatives Selektieren fetaler Zellen
umfasst.

12. Verfahren zum Analysieren des Genotyps einer fetalen Zelle an einer Stelle von Interesse, wobei das Verfahren
i) Verarbeiten einer transzervikalen Probe unter Verwendung eines Verfahrens gemäß einem der Ansprüche 7 bis 10 und/oder Anreichern fetaler Zellen unter Verwendung eines Verfahrens gemäß einem der Ansprüche 1 bis 6 oder 11 und
ii) Analysieren des Genotyps wenigstens einer fetalen Zellen an einer Stelle von Interesse
umfasst.

13. Verfahren zum Bestimmen des Geschlechts eines Fetus, wobei das Verfahren
i) Verarbeiten einer transzervikalen Probe unter Verwendung eines Verfahrens gemäß einem der Ansprüche 7 bis 10 und/oder Anreichern fetaler Zellen unter Verwendung eines Verfahrens gemäß einem der Ansprüche 1 bis 6 oder 11 und
ii) Analysieren wenigstens einer fetalen Zelle, um das Geschlecht des Fetus zu bestimmen,
umfasst.

14. Verfahren zum Bestimmen des Vaters eines Fetus, wobei das Verfahren
i) Verarbeiten einer transzervikalen Probe unter Verwendung eines Verfahrens gemäß einem der Ansprüche 7 bis 10 und/oder Anreichern fetaler Zellen unter Verwendung eines Verfahrens gemäß einem der Ansprüche 1 bis 6 oder 11 und
ii) Bestimmen des Genotyps des Kandidatenvaters an einer oder mehreren Stellen,
iii) Bestimmen des Genotyps des Fetus an einer oder mehreren der Stellen und
iv) Vergleichen der Genotypen von ii) und iii), um die Wahrscheinlichkeit zu bestimmen, dass der Kandidatenvater der biologische Vater des Fetus ist,
umfasst.

## Revendications

1. Procédés d'enrichissement de cellules foetales multinucléées à partir d'un échantillon transcervical d'une femme enceinte, le procédé comprenant la sélection de cellules qui ont une taille comprise entre environ 40 µm et 150 µm.

2. Procédé de la revendication 1, le procédé comprenant le traitement de l'échantillon pour produire au moins une suspension monocellulaire partielle avant que les cellules soient sélectionnées.

3. Procédé de la revendication 1, qui comprend
i) la désagrégation mécanique au moins partielle de l'échantillon pour produire au moins une suspension monocellulaire partielle,
ii) la filtration de l'au moins une suspension monocellulaire partielle à travers un premier filtre à cellules qui a une taille de maille d'environ 100 µm et la collecte des cellules qui traversent le premier filtre à cellules, et
iii) la filtration des cellules collectées dans l'étape ii) à travers un deuxième filtre à cellules qui a une taille de maille d'environ 40 µm et la collecte des cellules qui ne traversent pas le deuxième filtre à cellules.

4. Procédé de la revendication 1 qui comprend
i) la désagrégation enzymatique au moins partielle de l'échantillon pour produire au moins une suspension monocellulaire partielle, et
ii) la filtration de l'au moins une suspension monocellulaire partielle à travers un filtre à cellules qui a une taille de maille d'environ 40 µm et la collecte des cellules qui traversent le filtre à cellules.

5. Procédé de la revendication 1 qui comprend
i) la désagrégation mécanique au moins partielle de l'échantillon pour produire au moins une suspension monocellulaire partielle,
ii) la filtration de l'au moins une suspension monocellulaire partielle à travers un filtre à cellules qui a une taille de maille d'environ 100 µm et la collecte des cellules qui traversent le premier filtre à cellules, et
iii) le tri des cellules collectées dans l'étape ii) par séparation de cellules activée par fluorescence (FACS) basée sur la diffusion directe et la collecte des cellules qui ont une taille d'au moins environ 40 µm.

6. Procédé de la revendication 1, qui comprend
i) le traitement de l'échantillon pour produire au moins une suspension monocellulaire partielle, et
ii) le tri de l'au moins une suspension monocellulaire partielle par séparation de cellules activée par fluorescence (FACS) basée sur la diffusion directe et la collecte des cellules qui ont une taille comprise entre environ 40 µm et 100 µm.

7. Procédé pour traiter un échantillon transcervical d'une femme enceinte, le procédé comprenant
i) le traitement de l'échantillon pour produire au moins une suspension monocellulaire partielle,
ii) la sélection des cellules qui ont une taille comprise entre environ 40 µm et 150 µm,
dans lequel au moins une partie des cellules obtenues dans l'étape ii) sont des cellules foetales qui sont adaptées pour analyse génétique et/ou enrichissement.

8. Procédé de la revendication 7, dans lequel l'étape i) comprend le traitement de l'échantillon par désagrégation mécanique au moins partielle de l'échantillon, et/ou traitement de l'échantillon par désagrégation enzymatique au moins partielle de l'échantillon.

9. Procédé de la revendication 7 ou la revendications 8, dans lequel l'étape ii) comprend la filtration du produit de l'étape i) à travers un filtre à cellules et la collecte des cellules qui traversent le filtre à cellules.

10. Procédé de la revendication 9 qui comprend en outre
iii) le traitement du matériau qui ne traverse pas le filtre à cellules avec au moins une enzyme capable de dissocier les cellules agrégées, et
iv) la sélection des cellules obtenues dans l'étape iii) sur la base de la taille de cellule.

11. Procédés d'enrichissement de cellules foetales à partir d'un échantillon transcervical d'une femme enceinte, le procédé comprenant
i) le traitement d'un échantillon transcervical selon le procédé de l'une quelconque des revendications 7 à 10, et
ii) la sélection positive et/ou négative des cellules foetales.

12. Procédé pour analyser le génotype d'une cellule foetale à un locus d'intérêt, le procédé comprenant
i) le traitement d'un échantillon transcervical au moyen d'un procédé selon l'une quelconque des revendications 7 à 10 et/ou l'enrichissement des cellules foetales au moyen d'un procédé selon l'une quelconque des revendications 1 à 6 ou 11, et
ii) l'analyse du génotype d'au moins une cellule foetale à un locus d'intérêt.

13. Procédé de détermination du sexe d'un foetus, le procédé comprenant
i) le traitement d'un échantillon transcervical au moyen d'un procédé selon l'une quelconque des revendications 7 à 10 et/ou l'enrichissement des cellules foetales au moyen d'un procédé selon l'une quelconque des revendications 1 à 6 ou 11, et
ii) l'analyse d'au moins une cellule foetale pour déterminer le sexe du foetus.

14. Procédé de détermination du père d'un foetus, le procédé comprenant
i) le traitement d'un échantillon transcervical au moyen d'un procédé selon l'une quelconque des revendications 7 à 10 et/ou l'enrichissement des cellules foetales au moyen d'un procédé selon l'une quelconque des revendications 1 à 6 ou 11, et
ii) la détermination du génotype du père candidats à au moins un ou plusieurs locus,
iii) la détermination du génotype du foetus à un ou plusieurs desdits locus, et
iv) la comparaison des génotypes de ii) et ii) pour déterminer la probabilité que le père candidats soit le père biologique du foetus.
